# FASCICULE DE BREVET EUROPEEN

(11) **EP 0 788 492 B1**
(45) Date de publication et mention de la délivrance du brevet: **31.03.1999**
(21) Numéro de dépôt: 95935998.5
(22) Date de dépôt: 23.10.1995
(51) Int. Cl.: C07D 305/14, A61K 31/335

(54) **NOUVEAUX TAXOIDES, LEUR PREPARATION ET LES COMPOSITIONS PHARMACEUTIQUES QUI LES CONTIENNENT**
TEXOL-DERIVATE, DEREN HERSTELLUNG UND DIESE ENTHALTENDE PHARMAZEUTISCHE ZUSAMMENSTELLUNGEN
NOVEL TAXOIDS, PREPARATION THEREOF, AND PHARMACEUTICAL COMPOSITIONS CONTAINING SAME

(30) Priorité: 26.10.1994 FR 9412795
(43) Date de publication de la demande: 13.08.1997
(73) Titulaire: RHONE-POULENC RORER S.A., 92160 Antony (FR)
(72) Inventeur: BOUCHARD, Hervé, F-94200 Ivry-sur-Seine (FR); BOURZAT, Jean-Dominique, F-94300 Vincennes (FR); COMMER ON, Alain, F-94400 Vitry-sur-Seine (FR); TERRIER, Corinne, F-93190 Livry-Gargan (FR); ZUCCO, Martine, F-94320 Thiais (FR)
(74) Mandataire: Le Pennec, Magali
(86) Numéro de dépôt international: FR9501393
(87) Numéro de publication internationale: WO9613494

(56) Documents cités:
- WO-A-94/13654
- WO-A-94/13655
- WO-A-95/09163
- US-A- 5 254 580

## Description

La présente invention concerne de nouveaux taxoïdes de formule générale : 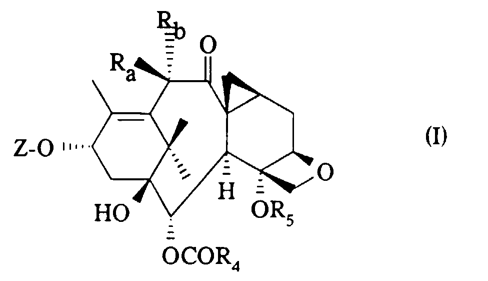 dans laquelle :
Rₐ représente un atome d'hydrogène ou un radical hydroxy, alcoxy contenant 1 à 4 atomes de carbone, acyloxy contenant 1 à 4 atomes de carbone ou alcoxyacétoxy dont la partie alcoyle contient 1 à 4 atomes de carbone et R_{b} représente un atome d'hydrogène ou bien Rₐ et R_{b} forment ensemble avec l'atome de carbone auquel ils sont liés une fonction cétone,
Z représente un atome d'hydrogène ou un radical de formule générale : 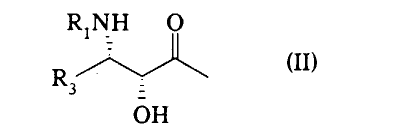 dans laquelle :
R₁ représente un radical benzoyle éventuellement substitué par un ou plusieurs atomes ou radicaux, identiques ou différents, choisis parmi les atomes d'halogène et les radicaux alcoyles contenant 1 à 4 atomes de carbone, alcoxy contenant 1 à 4 atomes de carbone ou trifluorométhyle, thénoyle ou furoyle, ou un radical R₂-O-CO- dans lequel R₂ représente :
   - un radical alcoyle contenant 1 à 8 atomes de carbone, alcényle contenant 2 à 8 atomes de carbone, alcynyle contenant 3 à 8 atomes de carbone, cycloalcoyle contenant 3 à 6 atomes de carbone, cycloalcényle contenant 4 à 6 atomes de carbone, bicycloalcoyle contenant 7 à 10 atomes de carbone, ces radicaux étant éventuellement substitués par un ou plusieurs substituants choisis parmi les atomes d'halogène et les radicaux hydroxy, alcoxy contenant 1 à 4 atomes de carbone, dialcoylamino dont chaque partie alcoyle contient 1 à 4 atomes de carbone, pipéridino, morpholino, pipérazinyl-1 (éventuellement substitué en -4 par un radical alcoyle contenant 1 à 4 atomes de carbone ou par un radical phénylalcoyle dont la partie alcoyle contient 1 à 4 atomes de carbone), cycloalcoyle contenant 3 à 6 atomes de carbone, cycloalcényle contenant 4 à 6 atomes de carbone, phényle (éventuellement substitué par un ou plusieurs atomes ou radicaux choisis parmi les atomes d'halogène et les radicaux alcoyles contenant 1 à 4 atomes de carbone ou alcoxy contenant 1 à 4 atomes de carbone), cyano, carboxy ou alcoxycarbonyle dont la partie alcoyle contient 1 à 4 atomes de carbone,
   - un radical phényle ou α- ou β-naphtyle éventuellement substitué par un ou plusieurs atomes ou radicaux choisis parmi les atomes d'halogène et les radicaux alcoyles contenant 1 à 4 atomes de carbone ou alcoxy contenant 1 à 4 atomes de carbone ou un radical hétérocyclique aromatique à 5 chaînons choisi de préférence parmi les radicaux furyle et thiényle,
   - ou un radical hétérocyclyle saturé contenant 4 à 6 atomes de carbone éventuellement substitué par un ou plusieurs radicaux alcoyles contenant 1 à 4 atomes de carbone,
R₃ représente un radical alcoyle droit ou ramifié contenant 1 à 8 atomes de carbone, alcényle droit ou ramifié contenant 2 à 8 atomes de carbone, alcynyle droit ou ramifié contenant 2 à 8 atomes de carbone, cycloalcoyle contenant 3 à 6 atomes de carbone, phényle ou α- ou β-naphtyle éventuellement substitué par un ou plusieurs atomes ou radicaux choisis parmi les atomes d'halogène et les radicaux alcoyles, alcényles, alcynyles, aryles, aralcoyles, alcoxy, alcoylthio, aryloxy, arylthio, hydroxy, hydroxyalcoyle, mercapto, formyle, acyle, acylamino, aroylamino, alcoxycarbonylamino, amino, alcoylamino, dialcoylamino, carboxy, alcoxycarbonyle, carbamoyle, alcoylcarbamoyle, dialcoylcarbamoyle, cyano, nitro et trifluorométhyle, ou un hétérocycle aromatique ayant 5 chaînons et contenant un ou plusieurs hétéroatomes, identiques ou différents, choisis parmi les atomes d'azote, d'oxygène ou de soufre et éventuellement substitué par un ou plusieurs substituants, identiques ou différents, choisis parmi les atomes d'halogène et les radicaux alcoyles, aryles, amino, alcoylamino, dialcoylamino, alcoxycarbonylamino, acyle, arylcarbonyle, cyano, carboxy, carbamoyle, alcoylcarbamoyle, dialcoylcarbamoyle ou alcoxycarbonyle, étant entendu que, dans les substituants des radicaux phényle, α- ou β-naphtyle et hétérocyclyles aromatiques, les radicaux alcoyles et les portions alcoyles des autres radicaux contiennent 1 à 4 atomes de carbone et que les radicaux alcényles et alcynyles contiennent 2 à 8 atomes de carbone et que les radicaux aryles sont des radicaux phényles ou α- ou β-naphtyles, et
R₄ représente
   - un radical alcoyle droit ou ramifié contenant 1 à 8 atomes de carbone, alcényle droit ou ramifié contenant 2 à 8 atomes de carbone, alcynyle droit ou ramifié contenant 2 à 8 atomes de carbone, cycloalcoyle contenant 3 à 6 atomes de carbone, cycloalcényle contenant 4 à 6 atomes de carbone ou bicycloalcoyle contenant 7 à 11 atomes de carbone, ces radicaux étant éventuellement substitués par un ou plusieurs substituants choisis parmi les atomes d'halogène et les radicaux hydroxy, alcoyloxy contenant -1 à 4 atomes de carbone, dialcoylamino dont chaque partie alcoyle contient 1 à 4 atomes de carbone, pipéridino, morpholino, pipérazinyl-1 (éventuellement substitué en -4 par un radical alcoyle contenant 1 à 4 atomes de carbone ou par un radical phénylalcoyle dont la partie alcoyle contient 1 à 4 atomes de carbone), cycloalcoyle contenant 3 à 6 atomes de carbone, cycloalcényle contenant 4 à 6 atomes de carbone, phényle éventuellement substitué, cyano, carboxy ou alcoyloxycarbonyle dont la partie alcoyle contient 1 à 4 atomes de carbone,
   - ou un radical aryle éventuellement substitué par un ou plusieurs atomes ou radicaux choisis parmi les atomes d'halogène et les radicaux alcoyles, alcényles, alcynyles, aryles, aralcoyles, alcoxy, alcoylthio, aryloxy, arylthio, hydroxy, hydroxyalcoyle, mercapto, formyle, acyle, acylamino, aroylamino, alcoxycarbonylamino, amino, alcoylamino, dialcoylamino, carboxy, alcoxycarbonyle, carbamoyle, alcoylcarbamoyle, dialcoylcarbamoyle, cyano, nitro, azido, trifluorométhyle ou trifluorométhoxy, ou un radical hétérocyclyle saturé ou non saturé contenant 4 à 6 chaînons et éventuellement substitué par un ou plusieurs radicaux alcoyles contenant 1 à 4 atomes de carbone, et
R₅ représente un radical alcoyle droit ou ramifié contenant 1 à 8 atomes de carbone, alcényle droit ou ramifié contenant 2 à 8 atomes de carbone, alcynyle droit ou ramifié contenant 2 à 8 atomes de carbone, cycloalcoyle contenant 3 à 6 atomes de carbone, cycloalcényle contenant 4 à 6 atomes de carbone ou bicycloalcoyle contenant 7 à 11 atomes de carbone, ces radicaux étant éventuellement substitués par un ou plusieurs substituants choisis parmi les atomes d'halogène et les radicaux hydroxy, alcoyloxy contenant 1 à 4 atomes de carbone, dialcoylamino dont chaque partie alcoyle contient 1 à 4 atomes de carbone, pipéridino, morpholino, pipérazinyl-1 (éventuellement substitué en -4 par un radical alcoyle contenant 1 à 4 atomes de carbone ou par un radical phénylalcoyle dont la partie alcoyle contient 1 à 4 atomes de carbone), cycloalcoyle contenant 3 à 6 atomes de carbone, cycloalcényle contenant 4 à 6 atomes de carbone, phényle éventuellement substitué, cyano, carboxy ou alcoyloxycarbonyle dont la partie alcoyle contient 1 à 4 atomes de carbone, étant entendu que les radicaux cycloalcoyles, cycloalcényles ou bicycloalcoyles peuvent être éventuellement substitués par un ou plusieurs radicaux alcoyles contenant 1 à 4 atomes de carbone.

De préférence les radicaux aryles pouvant être représentés par R₃ et/ou R₄ sont des radicaux phényles ou α- ou β-naphtyles éventuellement substitués par un ou plusieurs atomes ou radicaux choisis parmi les atomes d'halogène (fluor, chlore, brome, iode) et les radicaux alcoyles, alcényles, alcynyles, aryles, arylalcoyles, alcoxy, alcoylthio, aryloxy, arylthio, hydroxy, hydroxyalcoyle, mercapto, formyle, acyle, acylamino, aroylamino, alcoxycarbonylamino, amino, alcoylamino, dialcoylamino, carboxy, alcoxycarbonyle, carbamoyle, dialcoylcarbamoyle, cyano, nitro, azido, trifluorométhyle et trifluorométhoxy, étant entendu que les radicaux alcoyles et les portions alcoyles des autres radicaux contiennent 1 à 4 atomes de carbone, que les radicaux alcényles et alcynyles contiennent 2 à 8 atomes de carbone et que les radicaux aryles sont des radicaux phényles ou α- ou β-naphtyles.

De préférence les radicaux hétérocycliques pouvant être représentés par R₃ et/ou R₄ sont des radicaux hétérocycliques aromatiques ayant 5 chaînons et contenant un ou plusieurs atomes, identiques ou différents, choisis parmi les atomes d'azote, d'oxygène ou de soufre, éventuellement substitués par un ou plusieurs substituants, identiques ou différents, choisis parmi les atomes d'halogène (fluor, chlore, brome, iode) et les radicaux alcoyles contenant 1 à 4 atomes de carbone, aryles contenant 6 à 10 atomes de carbone, alcoxy contenant 1 à 4 atomes de carbone, aryloxy contenant 6 à 10 atomes de carbone, amino, alcoylamino contenant 1 à 4 atomes de carbone, dialcoylamino dont chaque partie alcoyle contient 1 à 4 atomes de carbone, acylamino dont la partie acyle contient 1 à 4 atomes de carbone, alcoxycarbonylamino contenant 1 à 4 atomes de carbone, acyle contenant 1 à 4 atomes de carbone, arylcarbonyle dont la partie aryle contient 6 à 10 atomes de carbone, cyano, carboxy, carbamoyle, alcoylcarbamoyle dont la partie alcoyle contient 1 à 4 atomes de carbone, dialcoylcarbamoyle dont chaque partie alcoyle contient 1 à 4 atomes de carbone ou alcoxycarbonyle dont la partie alcoxy contient 1 à 4 atomes de carbone.

Le document publié sous le numéro WO 94/13654 décrit des taxoïdes porteurs en position 7,8 d'un motif cyclopropyle, en position 4 d'un motif acétoxy, en position 2 d'un motif benzoyloxy présentant une activité antitumorale.

Le document publié sous le numéro WO 94/13655 décrit des taxoïdes porteurs en position 7,8 d'un motif cyclopropyle ou en position 7 d'un atome de fluor, en position 4 d'un motif acétoxy, en position 2 d'un motif benzoyloxy et sur la chaîne latérale porteurs en position 3' de divers motifs carbonylamino, en position 2' de motifs esters ou aminocarbonyl.

Le document publié sous le numéro US 5 254 580 décrit des taxoïdes porteurs en position 7,8 d'un motif cyclopropyl, en position 4 d'un motif acétoxy, en position 2 d'un motif benzoyloxy qui présentent des propriétés antitumorales.

Aucun de ces documents ne décrit de dérivés des taxoïdes porteurs en position 4 d'un motif éther objet de la présente invention.

Plus particulièrement, la présente invention concerne les produits de formule générale (I) dans laquelle Rₐ représente un radical hydroxy, alcoxy contenant 1 à 4 atomes de carbone, acyloxy contenant 1 à 4 atomes de carbone ou alcoxyacétoxy dont la partie alcoyle contient 1 à 4 atomes de carbone et R_{b} représente un atome d'hydrogène, Z représente un atome d'hydrogène ou un radical de formule générale (II) dans laquelle R₁ représente un radical benzoyle ou un radical R₂-O-CO- dans lequel R₂ représente un radical tert-butyle et R₃ représente un radical alcoyle contenant 1 à 6 atomes de carbone, alcényle contenant 2 à 6 atomes de carbone, cycloalcoyle contenant 3 à 6 atomes de carbone, phényle éventuellement substitué par un ou plusieurs atomes ou radicaux, identiques ou différents choisis parmi les atomes d'halogène (fluor, chlore) et les radicaux alcoyles (méthyle), alcoxy (méthoxy), dialcoylamino (diméthylamino), acylamino (acétylamino), alcoxycarbonylamino (tert-butoxycarbonylamino) ou trifluorométhyle ou un radical furyle-2 ou -3, thiényle-2 ou -3 ou thiazolyle-2, -4 ou -5 et R₄ représente un radical phényle éventuellement substitué par un ou plusieurs atomes ou radicaux, identiques ou différents, choisis parmi les atomes d'halogène et les radicaux alcoyles, alcoxy, amino, alcoylamino, dialcoylamino, acylamino, alcoxycarbonylamino, azido, trifluorométhyle et trifluorométhoxy, ou un radical thiényle-2 ou -3 ou furyle-2 ou -3 et R₅ représente un radical alcoyle éventuellement substitué contenant 1 à 4 atomes de carbone.

Plus particulièrement encore, la présente invention concerne les produits de formule générale (I) dans laquelle Rₐ représente un atome d'hydrogène ou un radical hydroxy ou acétyloxy ou méthoxyacétoxy et R_{b} représente un atome d'hydrogène, Z représente un atome d'hydrogène ou un radical de formule générale (II) dans laquelle R₁ représente un radical benzoyle ou un radical R₂-O-CO- dans lequel R₂ représente un radical tert-butyle et R₃ représente un radical isobutyle, isobutényle, butényle, cyclohexyle, phényle, furyle-2, furyle-3, thiényle-2, thiényle-3, thiazolyle-2, thiazolyle-4 ou thiazolyle-5 et R₄ représente un radical phényle éventuellement substitué par un atome d'halogène et R₅ représente un radical alcoyle contenant 1 à 4 atomes de carbone.

Les produits de formule générale (I) dans laquelle Z représente un radical de formule générale (II) présentent des propriétés antitumorales et antileucémiques remarquables.

Selon la présente invention, les produits de formule générale (I) dans laquelle Rₐ représente un atome d'hydrogène ou un radical alcoxy, acyloxy ou alcoxyacétoxy, R_{b} représente un atome d'hydrogène, R₄, R₅ et Z sont définis comme précédemment peuvent être obtenus par action d'un halogénure de métal alcalin (chlorure de sodium, iodure de sodium, fluorure de potassium) ou d'un azoture de métal alcalin (azoture de sodium) ou d'un sel d'ammonium quaternaire ou d'un phosphate de métal alcalin sur un produit de formule générale : 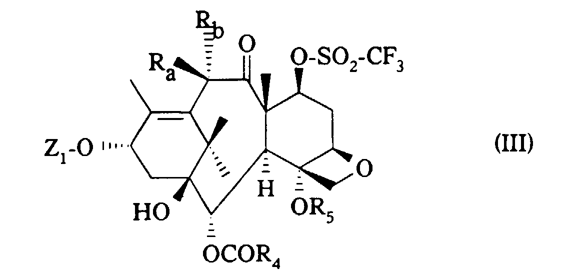 dans laquelle Z₁ représente un atome d'hydrogène ou un radical de formule générale (II) dans laquelle R₁ et R₃ sont définis comme précedemment ou un radical de formule générale : 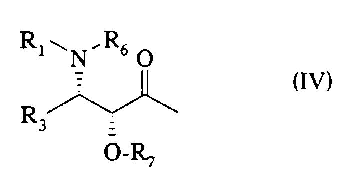 dans laquelle R₁ et R₃ sont définis comme précedemment, et, ou bien R₆ représente un atome d'hydrogène et R₇ représente un groupement protecteur de la fonction hydroxy, ou bien, R₆ et R₇ forment ensemble un hétérocycle saturé à 5 ou 6 chaînons, R₄ et R₅ sont définis comme précédemment, Rₐ représente un atome d'hydrogène ou un radical alcoxy, acyloxy, alcoxyacétoxy ou un radical hydroxy protégé, de préférence un radical trichloro-2,2,2 éthoxycarbonyloxy, et R_{b} représente un atome d'hydrogène, ou bien Rₐ et R_{b} forment ensemble avec l'atome de carbone auquel ils sont liés une fonction cétone, pour obtenir un produit de formule générale : 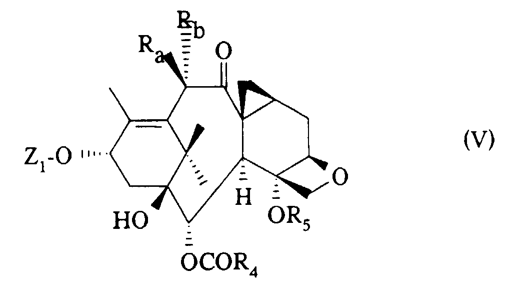 dans laquelle Z₁, R₄, R₅, Rₐ et R_{b} sont définis comme précédemment, suivi, si nécessaire, du remplacement du groupement protecteur porté par Rₐ ou des groupements protecteurs représentés par R₇ et/ou par R₆ et R₇ par des atomes d'hydrogène.

Généralement, la réaction est effectuée dans un solvant organique choisi parmi les éthers (tétrahydrofuranne, diisopropyléther, méthyl tert-butyléther) et les nitriles (acétonitrile) seul ou en mélange à une température comprise entre 20°C et la température d'ébullition du mélange réactionnel.

Un produit de formule générale (V), dans laquelle Z₁ représente un atome d'hydrogène ou un radical de formule générale (II), Rₐ représente un atome d'hydrogène ou un radical hydroxy, alcoxy contenant 1 à 4 atomes de carbone, acyloxy contenant 1 à 4 atomes de carbone ou alcoxyacétoxy dont la partie alcoyle contient 1 à 4 atomes de carbone et R_{b} représente un atome d'hydrogène ou bien Rₐ et R_{b} forment ensemble avec l'atome de carbone auquel ils sont liés une fonction cétone, est identique à un produit de formule générale (I).

Dans la formule générale (V), lorsque Z₁ représente un radical de formule générale (IV) et lorsque R₆ représente un atome d'hydrogène, R₇ représente de préférence un radical méthoxyméthyle, éthoxy-1 éthyle, benzyloxyméthyle, triméthylsilyle, triéthylsilyle, β-triméthylsilyléthoxyméthyle, benzyloxycarbonyle ou tétrahydropyrannyle ou bien lorsque R₆ et R₇ forment ensemble un hétérocycle, celui-ci est de préférence un cycle oxazolidine éventuellement mono-substitué ou gem-disubstitué en position -2.

Le remplacement des groupements protecteurs R₇ et/ou R₆ et R₇ par des atomes d'hydrogène et éventuellement de Rₐ par un radical hydroxy peut être effectué, selon leur nature de la manière suivante :
1) lorsque R₆ représente un atome d'hydrogène et R₇ représente un groupement protecteur de la fonction hydroxy, Rₐ représente un radical alcoxy, acyloxy ou alcoxyacétoxy, le remplacement des groupements protecteurs par des atomes d'hydrogène s'effectue au moyen d'un acide minéral (acide chlorhydrique, acide sulfurique, acide fluorhydrique) ou organique (acide acétique, acide méthanesulfonique, acide trifluorométhanesulfonique, acide p.toluènesulfonique) utilisé seul ou en mélange en opérant dans un solvant organique choisi parmi les alcools, les éthers, les esters, les hydrocarbures aliphatiques, les hydrocarbures aliphatiques halogénés, les hydrocarbures aromatiques ou les nitriles à une température comprise entre -10 et 60°C,
2) lorsque R₆ représente un atome d'hydrogène et R₇ représente un groupement protecteur de la fonction hydroxy, Rₐ représente un radical trichloro-2,2,2 éthoxycarbonyloxy, le remplacement du groupement protecteur R₇ est effectué dans les conditions décrites ci-dessus sous 1) et celui de Rₐ par traitement par le zinc, éventuellement associé à du cuivre, en présence d'acide acétique à une température comprise entre 30 et 60°C ou au moyen d'un acide minéral ou organique tel que l'acide chlorhydrique ou l'acide acétique en solution dans un alcool aliphatique contenant 1 à 3 atomes de carbone (méthanol, éthanol, propanol, isopropanol) ou dans un ester aliphatique (acétate d'éthyle, acétate d'isopropyle acétate de n.butyle) en présence de zinc éventuellement associé à du cuivre,
3) lorsque R₆ et R₇ forment ensemble un hétérocycle saturé à 5 ou 6 chaînons et plus particulièrement un cycle oxazolidine de formule générale : 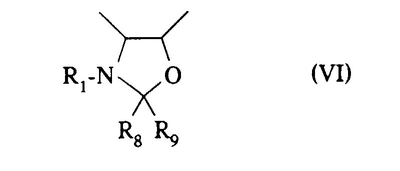 dans laquelle R₁ est défini comme précédemment, R₈ et R₉, identiques ou différents, représentent un atome d'hydrogène ou un radical alcoyle contenant 1 à 4 atomes de carbone, ou un radical aralcoyle dont la partie alcoyle contient 1 à 4 atomes de carbone et la partie aryle représente, de préférence, un radical phényle éventuellement substitué par un ou plusieurs radicaux alcoxy contenant 1 à 4 atomes de carbone, ou un radical aryle représentant, de préférence un radical phényle éventuellement substitué par un ou plusieurs radicaux alcoxy contenant 1 à 4 atomes de carbone, ou bien R₈ représente un radical alcoxy contenant 1 à 4 atomes de carbone ou un radical trihalométhyle tel que trichlorométhyle ou un radical phényle substitué par un radical trihalométhyle tel que trichlorométhyle et R₉ représente un atome d'hydrogène, ou bien R₈ et R₉ forment ensemble avec l'atome de carbone auquel ils sont liés un cycle ayant 4 à 7 chaînons, et Rₐ représente un radical acyloxy ou alcoxyacétoxy ou trichloro-2,2,2 éthoxycarbonyloxy, le remplacement du groupement protecteur formé par R₆ et R₇ par des atomes d'hydrogène et de Rₐ par un radical hydroxy peut être effectué, selon les significations de Rₐ, R₁, R₈ et R₉, de la manière suivante :
   a) lorsque R₁ représente un radical tert-butoxycarbonyle, R₈ et R₉, identiques ou différents, représentent un radical alcoyle ou un radical aralcoyle (benzyle) ou aryle (phényle), ou bien R₈ représente un radical trihalométhyle ou un radical phényle substitué par un radical trihalométhyle, et R₉ représente un atome d'hydrogène, ou bien R₈ et R₉ forment ensemble un cycle ayant de 4 à 7 chaînons, le traitement de l'ester de formule générale (V) par un acide minéral ou organique éventuellement dans un solvant organique tel qu'un alcool conduit au produit de formule générale : 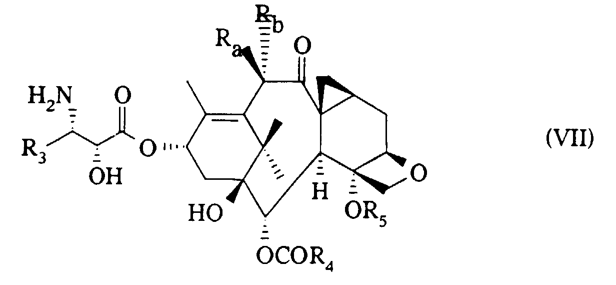 dans laquelle Rₐ, R_{b}, R₃, R₄ et R₅ sont définis comme précédemment, qui est acylé au moyen de chlorure de benzoyle dans lequel le noyau phényle est éventuellemnt substitué, de chlorure de thénoyle, de chlorure de furoyle ou d'un produit de formule générale :

      R₂-O-CO-X (VIII)

      dans laquelle R₂ est défini comme précédemment et X représente un atome d'halogène (fluor, chlore) ou un reste -O-R₂ ou -O-CO-O-R₂, pour obtenir un produit de formule générale : 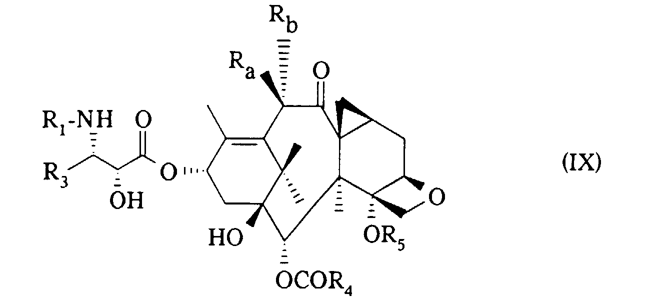 dans laquelle Rₐ, R_{b}, R₁, R₃, R₄ et R₅ sont définis comme précédemment, dont le groupement protecteur Rₐ, lorsqu'il représente un radical hydroxy protégé, est remplacé, si nécessaire, par un radical hydroxy.
      De préférence, le produit de formule générale (V) est traité par l'acide formique à une température voisine de 20°C.
      De préférence, l'acylation du produit de formule générale (IX) au moyen d'un chlorure de benzoyle dans lequel le radical phényle est éventuellement substitué, de chlorure de thénoyle ou de chlorure de furoyle ou d'un produit de formule générale (VIII) est effectuée dans un solvant organique inerte choisi parmi les esters tels que l'acétate d'éthyle, l'acétate d'isopropyle ou l'acétate de n.butyle et les hydrocarbures aliphatiques halogénés tels que le dichlorométhane ou le dichloro-1,2 éthane en présence d'une base minérale telle que le bicarbonate de sodium ou organique telle que la triéthylamine. La réaction est effectuée à une température comprise entre 0 et 50°C, de préférence voisine de 20°C.
      De préférence, le remplacement du groupement protecteur de Rₐ, lorsqu'il représente un radical trichloro-2,2,2 éthoxycarbonyloxy, est effectué dans les conditions décrites précédemment sous 2),
   b) lorsque R₁ représente un radical benzoyle éventuellement substitué, thénoyle ou furoyle ou un radical R₂O-CO- dans lequel R₂ est défini comme précédemment, R₈ représente un atome d'hydrogène ou un radical alcoxy contenant 1 à 4 atomes de carbone ou un radical phényle substitué par un ou plusieurs radicaux alcoxy contenant 1 à 4 atomes de carbone et R₉ représente un atome d'hydrogène, le remplacement du groupement protecteur formé par R₆ et R₇ par des atomes d'hydrogène s'effectue en présence d'un acide minéral (acide chlorhydrique, acide sulfurique) ou organique (acide acétique, acide méthane-sulfonique, acide trifluorométhanesulfonique, acide p.toluènesulfonique) utilisé seul ou en mélange en quantité stoechiométrique ou catalytique, en opérant dans un solvant organique choisi parmi les alcools, les éthers, les esters, les hydrocarbures aliphatiques, les hydrocarbures aliphatiques halogénés et les hydrocarbures aromatiques à une température comprise entre -10 et 60°C, de préférence entre 15 et 30°C et le remplacement du groupement protecteur de Rₐ, lorsqu'il représente un radical trichloro-2,2,2 éthoxycarbonyloxy par un atome d'hydrogène, s'effectue dans les conditions décrites précédemment sous 2).
4) lorsque Rₐ représente un radical alcoxyacétoxy et R₆ et R₇ sont définis comme au point 1) ci-dessus, on effectue d'abord le remplacement du groupement protecteur R₇ par un atome d'hydrogène en opérant dans les conditions acides décrites au point 1) ci-dessus, puis remplace éventuellement Rₐ par un radical hydroxy par traitement en milieu alcalin ou par action d'un halogénure de zinc dans des conditions qui ne touchent pas au reste de la molécule. Généralement, le traitement alcalin est effectué par action de l'ammoniac en milieu hydro-alcoolique ou de l'hydrate d'hydrazine en milieu alcoolique à une température voisine de 20°C. Généralement, le traitement par un halogénure de zinc, de préférence l'iodure de zinc, est effectué dans le méthanol à une température voisine de 20°C.
5) lorsque Rₐ représente un radical alcoxyacétoxy et R₆ et R₇ sont définis comme au point 3-a) ci-dessus, on effectue le remplacement du radical Rₐ par un radical hydroxy par traitement en milieu alcalin ou par traitement par un halogénure de zinc dans les conditions décrites au point 4) ci-dessus, puis traite le produit de formule générale (V) obtenu dans les conditions de déprotection et d'acylation décrites au point 3-a) ci-dessus.
6) lorsque Rₐ représente un radical alcoxyacétoxy et R₆ et R₇ sont définis comme au point 3-b) ci-dessus, on effectue le remplacement du radical Rₐ par un radical hydroxy par traitement en milieu alcalin ou par traitement par un halogénure de zinc dans les conditions décrites au point 4) ci-dessus, puis traite le produit obtenu dans les conditions décrites au point 3-b) ci-dessus.

Le produit de formule générale (III) dans laquelle Z₁ représente un radical de formule générale (II) ou un radical de formule générale (IV) peut être obtenu par estérification d'un produit de formule générale : 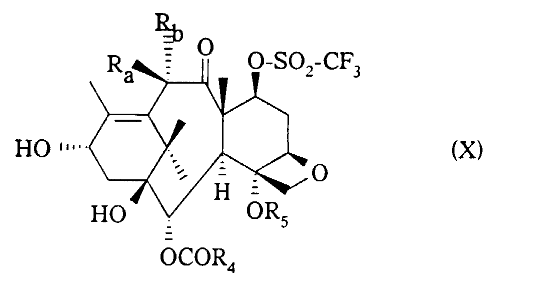 dans laquelle R₄, R₅ sont définis comme précédemment, et Rₐ représente un atome d'hydrogène ou un radical alcoxy, acyloxy, alcoxyacétoxy ou un radical hydroxy protégé, et R_{b} représente un atome d'hydrogène, au moyen d'un acide de formule générale : 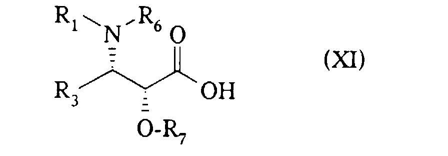 dans laquelle R₁ et R₃ sont définis comme précédemment, ou bien R₆ représente un atome d'hydrogène et R₇ représente un groupement protecteur de la fonction hydroxy, et ou bien R₆ et R₇ forment ensemble un hétérocycle saturé à 5 ou 6 chaînons, ou d'un dérivé de cet acide pour obtenir un ester de formule générale : 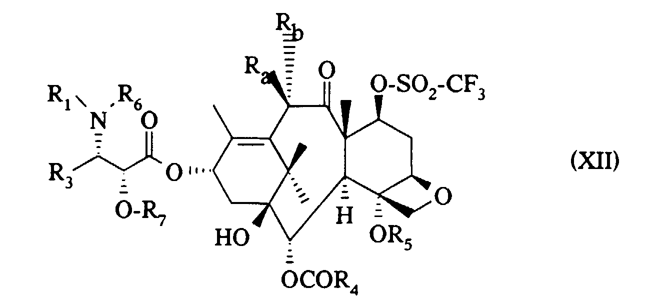 dans laquelle Rₐ, R_{b}, R₁, R₃, R₄, R₅, R₆ et R₇ sont définis comme précédemment, suivi, si nécessaire, du remplacement des groupements protecteurs représentés par R₇ et/ou R₆ et R₇ par des atomes d'hydrogène et éventuellement Rₐ, lorsqu'il représente un radical acyloxy, alcoxyacétoxy ou un radical hydroxy protégé, par un radical hydroxy dans les conditions décrites précédemment pour le remplacement des groupements protecteurs du produit de formule générale (V) dans laquelle Z₁ représente un radical de formule générale (IV).

L'estérification au moyen d'un acide de formule générale (XI) peut être effectuée en présence d'un agent de condensation (carbodiimide, carbonate réactif) et d'un agent d'activation (aminopyridines) dans un solvant organique (éther, ester, cétones, nitriles, hydrocarbures aliphatiques, hydrocarbures aliphatiques halogénés, hydrocarbures aromatiques) à une température comprise entre -10 et 90°C.

L'estérification peut aussi être réalisée en utilisant l'acide de formule générale (XI) sous forme d'anhydride en opérant en présence d'un agent d'activation (aminopyridines) dans un solvant organique (éthers, esters, cétones, nitriles, hydrocarbures aliphatiques, hydrocarbures aliphatiques halogénés, hydrocarbures aromatiques) à une température comprise entre 0 et 90°C.

L'estérification peut aussi être réalisée en utilisant l'acide de formule générale (XI) sous forme d'halogénure ou sous forme d'anhydride avec un acide aliphatique ou aromatique, éventuellement préparé in situ, en présence d'une base (amine aliphatique tertiaire) en opérant dans un solvant organique (éthers, esters, cétones, nitriles, hydrocarbures aliphatiques, hydrocarbures aliphatiques halogénés, hydrocarbures aromatiques) à une température comprise entre 0 et 80°C.

Selon l'invention, les produits de formule générale (III) dans laquelle R₄ et R₅ sont définis comme précédemment, Rₐ représente atome d'hydrogène ou un radical alcoxy, acyloxy ou alcoxyacétoxy, et R_{b} représente un atome d'hydrogène, et Z₁ représente un atome d'hydrogène peuvent être obtenus par action d'un dérivé de l'acide trifluorométhanesulfonique tel que l'anhydride ou le N-phényl trifluorométhanesulfonimide sur un produit de formule générale : 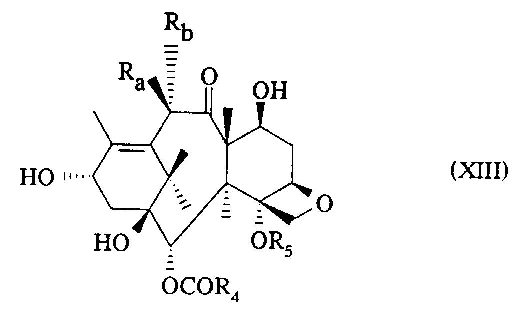 dans laquelle Rₐ, R_{b}, R₄ et R₅ sont définis comme précédemment.

Généralement, la réaction s'effectue dans un solvant organique inerte (hydrocarbures aliphatiques éventuellement halogénés, hydrocarbures aromatiques) en présence d'une base organique telle qu'une amine tertiaire aliphatique (triéthylamine) ou la pyridine à une température comprise entre -50 et +20°C.

Les produits de formule générale (XIII) dans laquelle R₄ et R₅ sont définis comme précédemment, Rₐ représente un atome d'hydrogène ou un radical alcoxy, acyloxy ou alcoxyacétoxy ou un radical hydroxy protégé, R_{b} représente un atome d'hydrogène, peuvent être obtenus par action de l'acide fluorhydrique ou de l'acide trifluoroacétique dans un solvant organique basique, tel que la pyridine éventuellement substituée par un ou plusieurs radicaux alcoyles contenant 1 à 4 atomes de carbone, ou la triéthylamine éventuellement en association avec un solvant organique inerte tel que le chlorure de méthylène ou l'acétonitrile ou le tétrahydrofuranne à une température comprise entre 20 et 80°C sur un produit de formule générale : 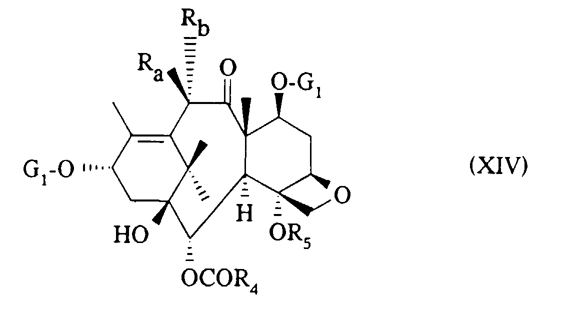 dans laquelle R₄ et R₅ sont définis comme précédemment, Rₐ représente un atome d'hydrogène ou un radical alcoxy, acyloxy ou alcoxyacétoxy ou un radical hydroxy protégé, R_{b} représente un atome d'hydrogène, et les symboles G₁, qui sont identiques représentent un radical trialcoylsilyle.

Le produit de formule générale (XIV), dans laquelle Rₐ représente un radical alcoxy, acyloxy ou alcoxyacétoxy ou un radical hydroxy protégé et R_{b} représente un atome d'hydrogène, peut être obtenu par action d'un produit de formule générale :

R-Y (XV)

dans laquelle R représente un radical alcoyle, alcanoyle ou alcoxyacétyle ou un groupement protecteur de la fonction hydroxy et Y représente un atome d'halogène sur un produit de formule générale : 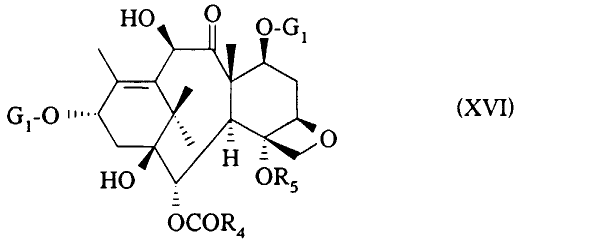 dans laquelle R₄, R₅ et G₁ sont définis comme précédemment.

Lorsque R représente un radical alcanoyle ou alcoxyacétyle il est particulièrement avantageux d'opérer dans un solvant organique basique tel que la pyridine ou dans un solvant organique inerte tel que le chlorure de méthylène, le chloroformée ou le dichloro-1,2 éthane en présence d'une amine tertiaire telle que la triéthylamine ou la pyridine à une température voisine de 0°C.

Lorsque R représente un radical alcoyle, il est particulièrement avantageux de métaller préalablement la fonction hydroxy en secondaire au moyen d'un hydrure alcalin (hydrure de sodium) ou d'un alcoylure métallique (butyllithium).

Le produit de formule générale (XVI) et éventuellement le produit de formule générale (XIV) peuvent être obtenus par action d'un dérivé organométallique de formule générale :

R₄-M (XVII)

dans laquelle R₄ est défini comme précédemment et M représente un atome métallique, de préférence un atome de lithium ou de magnésium, sur un produit de formule générale : 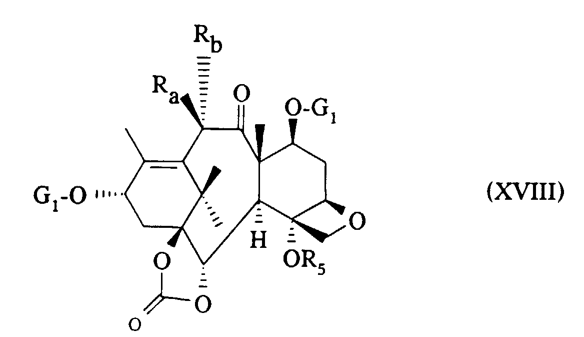 dans laquelle Rₐ, R_{b}, R₅ et G₁ sont définis comme précédemment.

Généralement, la réaction est effectuée dans un solvant organique tel qu'un éther (tétrahydrofuranne) à une température inférieure à -50°C, de préférence voisine de -78°C.

Le produit de formule générale (XVIII) peut être obtenu par ethérification d'un produit de formule générale : 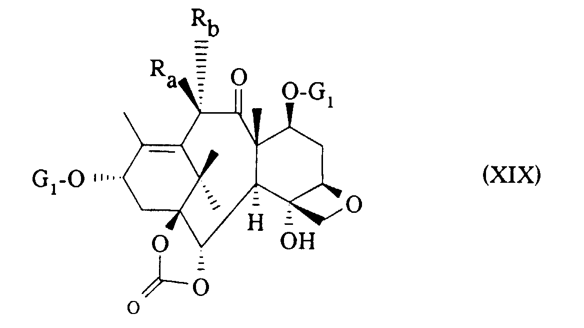 dans laquelle Rₐ, R_{b} et G₁ sont définis comme précédemment, au moyen d'un halogénure de formule générale :

R₅-Hal (XX)

dans laquelle R₅ est défini comme précédemment et Hal représente un atome d'halogène.

Il est particulièrement avantageux de métaller la fonction hydroxy tertiaire du produit de formule générale (XIX) par action d'un hydrure ou d'un amidure alcalin tel que l'hydrure de sodium ou le diisopropylamidure de lithium préalablement à l'action du produit de formule générale (XX).

Généralement la réaction est effectuée dans un solvant organique polaire tel que le diméthylformamide à une température comprise entre 0 et 50°C.

Le produit de formule générale (XIX) peut être obtenu par action d'un produit de formule générale (XV) sur un produit de formule générale : 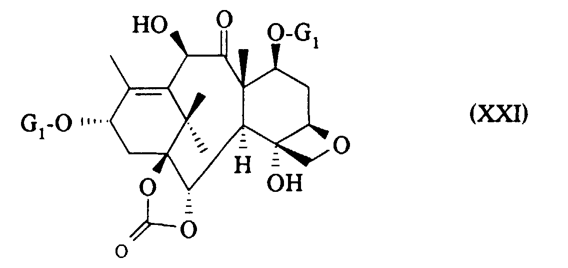 dans laquelle G₁ est défini comme précédemment dans les conditions décrites précédemment pour l'action d'un produit de formule générale (XV) sur un produit de formule générale (XVI).

Le produit de formule générale (XXI) peut être préparé par action du phosgène ou d'un de ses dérivés tel que le triphosgène sur un produit de formule générale : 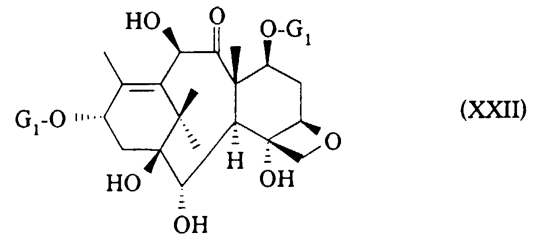 dans laquelle G₁ est défini comme précédemment en opérant dans un solvant organique basique tel que la pyridine à une température inférieure à -50°C, de préférence voisine de -78°C.

Le produit de formule générale (XXII) peut être préparé par action d'un halogénotrialcoylsilane sur un produit de formule générale : 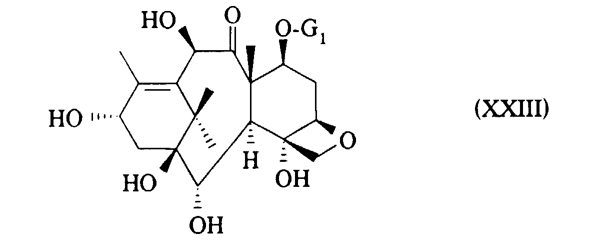 dans laquelle G₁ est défini comme précédemment en opérant dans un solvant organique basique.

Le produit de formule générale (XXIII) peut être préparé dans les conditions décrites par D.G.I. Kingston et coll., Journal of Nat. Prod., 56, 884 (1993).

Les produits de formule générale (XIV) dans laquelle R₄ représente un radical phényle, R₅ est défini comme précédemment, Rₐ représente un atome d'hydrogène ou un radical hydroxy, alcoxy, acyloxy ou alcoxyacétoxy ou un radical hydroxy protégé et R_{b} représente un atome d'hydrogène peut être obtenus par action d'un produit de formule générale (XX) sur un produit de formule générale : 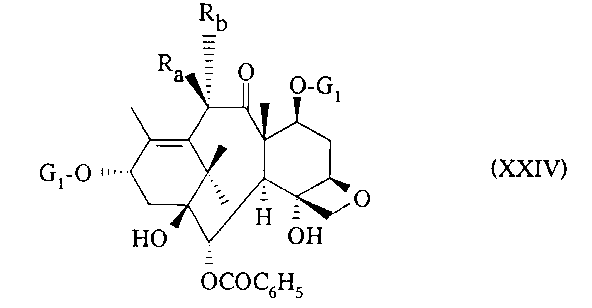 dans laquelle Rₐ, R_{b} sont définis comme ci-dessus et G₁ est défini comme précédemment, dans les conditions décrites ci-dessus pour l'action d'un produit de formule générale (XX) sur un produit de formule générale (XIX).

Les produits de formule générale (XXIV) dans laquelle Rₐ représente un groupement protecteur de la fonction hydroxy identique à G₁ et R_{b} représente un atome d'hydrogène peuvent être obtenus par action d'un halogénotrialcoylsilane sur un produit de formule générale : 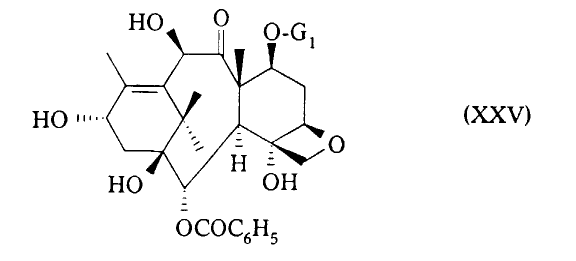 dans laquelle G₁ est défini comme précédemment.

La réaction est effectuée de préférence dans un solvant organique tel que le diméthylformamide en présence d'imidazole.

Les produits de formule générale (XXIV), dans laquellle Rₐ représente un radical alcoxy, acyloxy ou alcoxyacétoxy, R_{b} représente un atome d'hydrogène et G₁ est défini comme précédemment, peuvent être obtenus par action d'un produit de foormule générale (XV) sur un produit de formule générale : 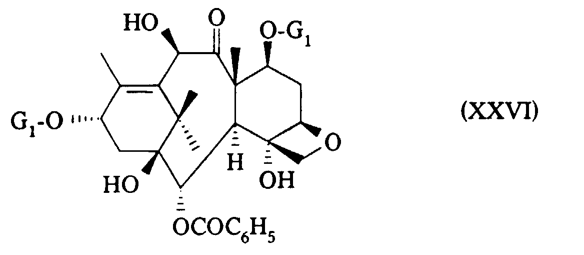 dans laquelle G₁ est défini comme précédemment, dans les conditions décrites précédemment pour l'action d'un produit de formule générale (XV) sur un produit de formule générale (XVI).

Les produits de formule générale (XXVI) peuvent être obtenus par action d'un halogénotrialcoylsilane sur un produit de formule générale (XXV) dans les conditions décrites précedemment pour l'action d'un halogénotrialcoylsilane sur un produit de formule générale (XXIII).

Les produits de formule générale (XXV) peuvent être obtenus dans les conditions décrites par D. G. I. Kingston et coll., Tetrahedron Letters, 35, 6839 (1992).

Les produits de formule générale (I) dans laquelle Rₐ et R_{b} représentent chacun un atome d'hydrogène peuvent être obtenus par réduction électrolytique d'un produit de formule générale (I) dans laquelle Rₐ représente un radical hydroxy ou un radical acyloxy ou alcoxyacétoxy ou dans les conditions décrites dans la demande internationale PCT WO 93/06093.

Les produits de formule générale (I) dans laquelle Rₐ et R_{b} forment ensemble avec l'atome de carbone auquel ils sont liés une fonction cétone peuvent être obtenus par oxydation d'un produit de formule générale (I) dans laquelle Rₐ représente un radical hydroxy et R_{b} représente un atome d'hydrogène au moyen, par exemple, de chlorochromate de pyridinium, de dichromate de pyridinium, de bichromate de potassium, de bichromate d'ammonium ou de bioxyde de manganèse.

Les nouveaux produits de formule générale (I) obtenus par la mise en oeuvre des procédés selon l'invention peuvent être purifiés selon les méthodes connues telles que la cristallisation ou la chromatographie.

Les produits de formule générale (I) dans laquelle Z représente un radical de formule générale (II) présentent des propriétés biologiques remarquables.

In vitro, la mesure de l'activité biologique est effectuée sur la tubuline extraite de cerveau de porc par la méthode de M.L. Shelanski et coll., Proc. Natl. Acad. Sci. USA, 70, 765-768 (1973). L'étude de la dépolymérisation des microtubules en tubuline est effectuée selon la méthode de G. Chauvière et coll., C.R. Acad. Sci., 293, série II, 501-503 (1981). Dans cette étude les produits de formule générale (I) dans laquelle Z représente un radical de formule générale (II) se sont montrés au moins aussi actifs que le taxol et le Taxotère.

In vivo, les produits de formule générale (I) dans laquelle Z représente un radical de formule générale (II) se sont montrés actifs chez la souris greffée par le mélanome B16 à des doses comprises entre 1 et 10 mg/kg par voie intrapéritonéale, ainsi que sur d'autres tumeurs liquides ou solides.

Les nouveaux produits ont des propriétés anti-tumorales et plus particulièrement une activité sur les tumeurs qui sont résistantes au Taxol® ou au Taxotère®. De telles tumeurs comprennent les tumeurs du colon qui ont une expression élevée du gène mdr 1 (gène de la multi-drug resistance). La multi-drug resistance est un terme habituel se rapportant à la résistance d'une tumeur à différents produits de structures et de mécanismes d'action différents. Les taxoïdes sont généralement connus pour être fortement reconnus par des tumeurs expérimentales telles que P388/DOX, une lignée cellulaire sélectionnée pour sa résistance à la doxorubicine (DOX) et qui surexprime mdr 1.

Les exemples suivants illustrent la présente invention.

### EXEMPLE 1

Une solution de 40 mg de tert-butoxycarbonyl-3 (méthoxy-4 phényl)-2 phényl-4 oxazolidine-1,3 carboxylate-5-(2R,4S,5R) de benzoyloxy-2α dihydroxy-1β,10β époxy-5β,20 méthoxy-4α méthylène-7β,8 nor-19 oxo-9 taxène-11 yle-13α dans 450 µl d'une solution 0,1N d'éthanol chlorhydrique est maintenue sous agitation à une température voisine de 0°C pendant 3 heures. Le mélange réactionnel est concentré à sec sous pression réduite (2,7 kPa) à 20°C. Le produit brut obtenu est dissous dans 10 cm3 de dichlorométhane et 10 cm3 d'une solution aqueuse saturée de bicarbonate de sodium. La phase organique est séparée par décantation lavée par 2 fois 10 cm3 d'une solution aqueuse saturée de chlorure de sodium puis séchée sur sulfate de magnésium, filtrée et concentrée à sec sous pression réduite (2,7 kPa) à 40°C. On obtient 59 mg d'un produit que l'on purifie par chromatographie préparative sur plaque de silice de 0,5 mm d'épaisseur en éluant avec un mélange cyclohexane-acétate d'éthyle (60-40 en volumes). On obtient ainsi 12 mg de tert-butoxycarbonylamino-3 hydroxy-2 phényl-3 propionate-(2R,3S) de benzoyloxy-2α dihydroxy-1β,10β époxy-5β,20 méthoxy-4α méthylène-7β,8 nor-19 oxo-9 taxène-11 yle-13α, sous forme d'une meringue blanche, dont les caractéristiques physiques sont les suivantes :
- spectre de R.M.N. du proton (400 MHz ; CDCl₃ ; δ en ppm ; constantes de couplage J en Hz) : 1,20 (s, 6H : CH₃) ; 1,35 (s, 9H : C(CH₃)₃ ; de 1,35 à 1,60 (mt, 1H : H en 7) ; 1,73 (s, 1H : OH en 1) ; 1,81 et 2,27 (2 mts, 1H chacun : CH₂ en 19) ; 1,90 (s, 3H : CH₃) ; 2,07 et 2,26 (2 mts, 1H chacun : CH₂ en 6) ; 2,35 et 2,87 (2 mts, 1H chacun: CH₂ en 14) ; 3,24 (mt, 1H : OH en 2') ; 3,46 (s, 3H : OCH₃) ; 3,70 (d, J = 7, 1H : H en 3) ; 3,97 et 4,39 (2 d, J = 9, 1H chacun : CH₂ en 20) ; 4,24 (s large, 1H : OH en 10) ; 4,62 (mt, 1H : H en 2') ; 4,90 (d large, J = 4, 1H : H en 5) ; 4,98 (s, 1H : H en 10) ; 5,36 (mt, 1H : H en 3') ; 5,48 (d, J = 10, 1H : CONH) ; 5,69 (d, J = 7, 1H : H en 2) ; 6,24 (mt, 1H : H en 13) ; de 7,25 à 7,50 (mt, 5H : H aromatiques en 3' ) ; 7,49 (t, J = 7,5, 2H : OCOC₆H₅ H en méta) ; 7,57 (t, J = 7,5, 1H : OCOC₆H₅ H en para) ; 8,12 (d, J = 7,5, 2H : OCOC₆H₅ H en ortho).

Le tert-butoxycarbonyl-3 (méthoxy-4 phényl)-2 phényl-4 oxazolidine-1,3 carboxylate-5-(2R,4S,5R) de benzoyloxy-2α dihydroxy-1β,10β époxy-5β,20 méthoxy-4α méthylène-7β,8 nor-19 oxo-9 taxène-11 yle-13α peut être préparé de la manière suivante :

A une solution de 112 mg de tert-butoxycarbonyl-3 (méthoxy-4 phényl)-2 phényl-4 oxazolidine-1,3 carboxylate-5-(2R,4S,5R) de benzoyloxy-2α dihydroxy-1β,10β époxy-5β,20 méthoxy-4α oxo-9 trifluorométhanesulfonyloxy-7β taxène-11 yle-13α dans 1 cm3 d'acétonitrile et 0,1 cm3 de tétrahydrofuranne, on ajoute successivement 100 mg de tamis moléculaire 4Å en poudre et 100 mg d'azoture de sodium. Le mélange réactionnel est maintenu sous agitation à une température voisine de 75°C pendant 3 heures puis, à une température voisine de 20°C, additionné de 50 cm3 de dichlorométhane et 50 cm3 d'une solution aqueuse saturée de chlorure de sodium. La phase organique est séparée par décantation, lavée par 2 fois 40 cm3 d'une solution aqueuse saturée de chlorure de sodium puis séchée sur sulfate de magnésium, filtrée et concentrée à sec sous pression réduite (2,7 kPa) à 40°C. On obtient 77 mg d'un produit que l'on purifie par chromatographie prépaprative sur plaque de silice de 2 mm d'épaisseur en éluant avec un mélange dichlorométhane-méthanol (90-10 en volumes). On obtient ainsi 45 mg de tert-butoxycarbonyl-3 (méthoxy-4 phényl)-2 phényl-4 oxazolidine-1,3 carboxylate-5-(2R,4S,5R) de benzoyloxy-2α dihydroxy-1β,10β époxy-5β,20 méthoxy-4α méthylène-7β,8 nor-19 oxo-9 taxène-11 yle-13α, sous forme d'une meringue blanche, dont les caractéristiques physiques sont les suivantes :
- spectre de R.M.N. du proton (400 MHz ; CDCl₃ ; à une température de 330°K ; δ en ppm ; constantes de couplage J en Hz) : 1,10 (s, 9H : C(CH₃)₃) ; 1,20 (s, 3H : CH₃) ; 1,22 (s, 3H : CH₃) ; 1,29 (mt, 1H : H en 7) ; 1,68 (s, 3H : CH₃) ; 1,78 et de 2,25 à 2,35 (2 mts, 1H chacun : CH₂ en 19) ; 1,99 et 2,24 (respectivement dt et d large, J = 17 et 4 et J = 17, 1H chacun : CH₂ en 6) ; de 2,25 à 2,35 et 2,56 (respectivement mt et dd (J = 15 et 7), 1H chacun : CH₂ en 14) ; 3,06 (s, 3H : OCH₃) ; 3,67 (d, J = 7, 1H : H en 3) ; 3,80 (s, 3H : ArOCH₃) ; 3,96 et 4,26 (2 d, J = 9, 1H chacun : CH₂ en 20) ; 4,17 (s large, 1H : OH en 10) ; 4,66 (d, J = 5, 1H : H en 2') ; 4,78 (d large, J = 4, 1H : H en 5) ; 4,92 (s large, 1H : H en 10) ; 5,48 (mt, 1H : H en 3') ; 5,66 (d, J = 7, 1H : H en 2) ; 6,05 (mt, 1H : H en 13) ; 6,38 (s, 1H : H en 5') ; 6,91 (d, J = 8,5, 2H : H aromatiques en ortho du OCH₃) ; de 7,30 à 7,50 (mt, 7H : H aromatiques en 3' et OCOC₆H₅ H en méta) ; 7,41 (d, J = 8,5, 2H : H aromatiques en méta du OCH₃) ; 7,58 (t, J = 7,5, 1H : OCOC₆H₅ H en para) ; 7,96 (d, J = 7,5, 2H : OCOC₆H₅ H en ortho).

Le tert-butoxycarbonyl-3 (méthoxy-4 phényl)-2 phényl-4 oxazolidine-1,3 carboxylate-5-(2R,4S,5R) de benzoyloxy-2α dihydroxy-1β,10β époxy-5β,20 méthoxy-4α oxo-9 trifluorométhanesulfonyloxy-7β taxène-11 yle-13α peut être préparé de la manière suivante :

A une solution de 100 mg de benzoyloxy-2α époxy-5β,20 méthoxy-4α oxo-9 trifluorométhanesulfonyloxy-7β trihydroxy-1β,10β,13α taxène-11 dans 4 cm3 d'acétate d'éthyle anhydre, on ajoute successivement 70 mg d'acide tert-butoxycarbonyl-3 (méthoxy-4 phényl)-2 phényl-4 oxazolidine-1,3 carboxylique-5-(2R,4S,5R), 50 mg de dicydohexylcarbodiimide et 6 mg de diméthylammo-4 pyridine à une température voisine de 20°C. Le mélange réactionnel est agité pendant 3 heures 30 minutes, sous atmosphère d'argon, à une température voisine de 20°C. On ajoute 30 cm3 d'acétate d'éthyle et 20 cm3 d'une solution aqueuse saturée de chlorure d'ammonium. La phase organique est séparée par décantation, lavée par 2 fois 20 cm3 d'eau puis séchée sur sulfate de magnésium. Après filtration et concentation à sec sous pression réduite (2,7 kPa) à 40°C, on obtient 200 mg d'un produit que l'on purifie par chromatographie sur 15 g de silice (0,063-0,2 mm) contenus dans une colonne de 1 cm de diamètre en éluant avec un mélange cyclohexane-acétate d'éthyle (80-20 en volumes) en recueillant des fractions de 8 cm3. Les fractions ne contenant que le produit cherché sont réunies et concentrées à sec sous pression réduite (2,7 kPa) à 40°C. On obtient ainsi 112 mg de tert-butoxycarbonyl-3 (méthoxy-4 phényl)-2 phényl-4 oxazolidine-1,3 carboxylate-5-(2R,4S,5R) de benzoyloxy-2α dihydroxy-1β,10β époxy-5β,20 méthoxy-4α oxo-9 trifluorométhanesulfonyloxy-7β taxène-11 yle-13α, sous forme d'une meringue blanche, dont les caractéristiques physiques sont les suivantes :
- spectre de R.M.N. du proton (400 MHz ; CDCl₃, à une température de 333°K, δ en ppm ; constantes de couplage J en Hz) : 1,10 (s, 9H : C(CH₃)₃) ; 1,13 (s, 3H : CH₃) ; 1,18 (s, 3H : CH₃) ; 1,77 (s, 3H: CH₃) ; 1,87 (s, 3H : CH₃) ; 2,33 et 2,76 (2 dd, J = 15 et 11 Hz et J = 15 et 7, 1H chacun : CH₂ en 14) ; 2,36 et 2,68 (2 mts, 1H chacun : CH₂ en 6) ; 3,19 (s, 3H : OCH₃) ; 3,44 (d, J = 6, 1H : H en 3) ; 3,83 (s, 3H : ArOCH₃) ; 3,95 (s large, 1H : OH en 10) ; 4,19 et 4,34 (2 d, J = 9, 1H chacun : CH₂ en 20) ; 4,66 (d, J = 5,5, 1H : H en 2') ; de 4,85 à 4,95 (mt, 2H : H en 7 et H en 5) ; 5,32 (s large, 1H : H en 10) ; 5,49 (d, J = 5,5, 1H : H en 3') ; 5,60 (d, J = 6, 1H : H en 2) ;5,95 (mt, 1H: H en 13) ; 6,38 (s, 1H : H en 5') ; 6,94 (d, J = 8,5, 2H : H aromatiques en ortho du OCH₃) ; de 7,30 à 7,50 (mt, 7H : H aromatiques en 3' et OCOC₆H₅ H en méta) ; 7,40 (d, J = 8,5, 2H : H aromatiques en méta du OCH₃) ; 7,60 (t, J = 7,5, 1H : OCOC₆H₅ H en para) ; 7,97 (d, J = 7,5, 2H : OCOC₆H₅ H en ortho).

Le benzoyloxy-2α époxy-5β,20 méthoxy-4α oxo-9 trifluorométhanesulfonyloxy-7β trihydroxy-1β,10β,13α taxène-11 peut être préparé de la manière suivante :

A une solution de 51,6 mg de benzoyloxy-2α époxy-5β,20 méthoxy-4α oxo-9 tétrahydroxy-1β,7β,10β,13α taxène-11 dans 0,5 cm3 de dichlorométhane et 24 µl de pyridine, on ajoute 25 µl d'anhydride triflique à une température voisine de 0°C. Le mélange réactionnel est agité pendant 20 minutes à une température voisine de 0°C puis on ajoute 15 cm3 de dichlorométhane et 3 cm3 d'eau. La phase organique est séparée par décantation, lavée par 2 fois 10 cm3 d'une solution aqueuse saturée de chlorure d'ammonium puis séchée sur sulfate de magnésium. Après filtration et concentation à sec sous pression réduite (2,7 kPa) à 20°C, on obtient 72 mg d'un produit que l'on purifie par chromatographie sur plaque de silice d'épaisseur de 2 mm en éluant avec un mélange dichlorométhane-méthanol (90-10 en volumes). On obtient ainsi 8 mg de benzoyloxy-2α époxy-5β,20 méthoxy-4α trihydroxy-1β,10β,13α oxo-9 trifluorométhanesulfonyloxy-7β taxène-11, sous forme d'une meringue blanche, dont les caractéristiques physiques sont les suivantes :
- spectre de R.M.N. du proton (400 MHz ; CDCl₃ ; δ en ppm ; constantes de couplage J en Hz) : 1,06 (s, 3H : CH₃) ; 1,10 (s, 3H : CH₃) ; 1,86 (s, 3H : CH₃) ; 2,13 (s, 3H : CH₃) ; 2,41 et 2,74 (2 mts, 1H chacun : CH₂ en 6) ; 2,47 et 2,59 (2 dd, J = 16 et 10 et J = 16 et 4, 1H chacun : CH₂ en 14) ; 2,96 (d large, J = 10, 1H : OH en 13) ; 3,66 (s, 3H : OCH₃) ; 3,79 (d, J = 6, 1H : H en 3) ; 3,95 (s large, 1H : OH en 10) ; 4,30 et 4,49 (2 d, J = 9, 1H chacun : CH₂ en 20) ; 4,57 (mt, 1H : H en 13); 4,98 (dd, J = 12 et 6, 1H : H en 7) ; 5,04 (dd, J = 10 et 3, 1H : H en 5) ; 5,42 (s large, 1H : H en 10) ; 5,61 (d, J = 6, 1H : H en 2) ; 7,59 (t, J = 7,5, 2H : OCOC₆H₅ H en métal) ; 7,63 (t, J = 7,5, 1H : OCOC₆H₅ H en para) ; 8,00 (d, J = 7,5, 2H : OCOC₆H₅ H en ortho).

Le benzoyloxy-2α époxy-5β,20 méthoxy-4α oxo-9 tétrahydroxy-1β,7β,10β,13α taxène-11 peut être préparé de la manière suivante :

A une solution de 338 mg de benzoyloxy-2α dihydroxy-1β,10β ditriéthylsilyloxy-7β,13α époxy-5β,20 méthoxy-4α oxo-9 taxène-11 dans 5 cm3 de dichlorométhane, on ajoute, à une température voisine de 20°C, 7,5 cm3 de complexe triéthylamine-acide fluorhydrique. Le mélange réactionnel est agité pendant 2 heures à une température voisine de 20°C puis on ajoute 50 cm3 de dichlorométhane et 50 cm3 d'une solution aqueuse saturée d'hydrogénocarbonate de sodium. La phase organique est séparée par décantation, lavée par 2 fois 50 cm3 d'une solution aqueuse saturée de chlorure de sodium puis séchée sur sulfate de magnésium. Après filtration et concentration à sec sous pression réduite (2,7 kPa) à 40°C, on obtient 420 mg d'un produit que l'on purifie par chromatographie sur 60 g de silice (0,063-0,2 mm) contenus dans une colonne de 1 cm de diamètre en éluant avec un mélange dichlorométhane-méthanol (95-5 en volumes) en recueillant des fractions de 10 cm3. Les fractions ne contenant que le produit cherché sont réunies et concentrées à sec sous pression réduite (2,7 kPa) à 40°C. On obtient ainsi 184 mg de benzoyloxy-2α époxy-5β,20 méthoxy-4α oxo-9 tétrahydroxy-1β,7β,10β,13α taxène-11, sous forme d'une meringue blanche, dont les caractéristiques physiques sont les suivantes :
- spectre de R.M.N.du proton (400 MHz ; CDCl₃ ; δ en ppm ; constantes de couplage J en Hz) : 1,05 (s, 3H : CH₃) ; 1,10 (s, 3H : CH₃) ; 1,72 (s, 3H : CH₃) ; 1,99 et de 2,40 à 2,55 (2 mts, 1H chacun : CH₂ en 6) ; 2,09 (s, 3H : CH₃) ; 2,48 et 2,69 (2 dd, J = 16 et 10 et J = 16 et 4, 1H chacun : CH₂ en 14) ; 3,15 (d large, J = 11, 1H : OH en 13) ; 3,65 (s, 3H : OCH₃) ; 3,74 (d, J = 6, 1H : H en 3) ; 3,78 (dd, J = 12 et 6, 1H : H en 7) ; 4,13 (s large, 1H : OH en 10) ; 4,31 et 4,45 (2 d, J = 9,5, 1H chacun : CH₂ en 20) ; 4,54 (mt, 1H : H en 13) ; 5,00 (dd, J = 10 et 3, 1H : H en 5) ; 5,27 (s large, 1H : H en 10) ; 5,61 (d, J = 6, 1H : H en 2) ; 7,48 (t, J = 7,5, 2H : OCOC₆H₅ H en méta) ; 7,62 (t, J = 7,5, 1H : OCOC₆H₅ H en para) ; 8,03 (d, J = 7,5, 2H : OCOC₆H₅ H en ortho).

Le benzoyloxy-2α dihydroxy-1β,10β ditriéthylsilyloxy-7β,13α époxy-5β,20 méthoxy-4α oxo-9 taxène-11 peut être préparé de la manière suivante:

A une solution de 940 mg de carbonate-1β,2α ditriéthylsilyloxy-7β,13α époxy-5β,20 méthoxy-4α méthoxyacétoxy-10β oxo-9 taxène-11 dans 45 cm3 de tétrahydrofuranne anhydride, on ajoute 2,22 cm3 d'une solution 1M de phényllithium dans le tétrahydrofurane à une température voisine de -78°C. Le mélange réactionnel est agité pendant 2 heures 30 minutes à une température voisine de -78°C puis on ajoute 20 cm3 d'une solution aqueuse saturée de chlorure d'ammonium. A une température voisine de 20°C, on ajoute 50 cm3 d'eau et 100 cm3 de d'acétate d'éthyle. La phase organique est séparée par décantation, lavée par 2 fois 50 cm3 d'une solution aqueuse saturée de chlorure de sodium puis séchée sur sulfate de magnésium. Après filtration et concentration à sec sous pression réduite (2,7 kPa) à 40°C, on obtient 1,2 g d'un produit que l'on purifie par chromatographie sur 100 g de silice (0,063-0,2 mm) contenus dans une colonne de 3 cm de diamètre en éluant avec un mélange acétate d'éthyle-cyclohexane (15-85 en volumes) en recueillant des fractions de 10 cm3. Les fractions ne contenant que le produit cherché sont réunies et concentrées à sec sous pression réduite (2,7 kPa) à 40°C. On obtient ainsi 734 mg de benzoyloxy-2α dihydroxy-1β,10β ditriéthylsilyloxy-7β,13α époxy-5β,20 méthoxy-4α oxo-9 taxène-11, sous forme d'une meringue blanche, dont les caractéristiques physiques sont les suivantes :
- spectre de R.M.N. proton (400 MHz ; CDCl₃, δ en ppm ; constantes de couplage J en Hz) : 0,55 (mt, 6 H : CH₂ éthyle) ; 0,70 (q, J = 7,5, 6 H : CH₂ éthyle) ; 0,93 (t, J = 7,5, 9H : CH₃ éthyle); 1,05 (t, J = 7,5, 9H : CH₃ éthyle) ; 1,09 (s, 3H : CH₃) ; 1,14 (s, 3H : CH₃) ; 1,55 (s, 1H : OH en 1) ; 1,71 (s, 3H : CH₃) ; 2,02 et 2,33 (2 mts, 1H chacun : CH₂ en 6) ; 2,07 (s, 3H : CH₃) ; 2,12 et 2,74 (2 dd, J = 15 et 9 Hz et J = 15 et 7,5, 1H chacun: CH₂ en 14) ; 3,43 (s, 3H : OCH₃) ; 3,47 (d, J = 7, 1H : H en 3) ; 3,85 (dd, = 11 et 6, 1H : H 7) ; 4,17 et 4,29 (2 d, = 8,5, 1H chacun : CH₂ 20) ; 4,27 (d, J = 2, 1H : OH en 10) ; 4,95 (mt, 1H : H en 13) ; 5,00 (dd, = 10 et 3, 1H : H en 5) ; 5,14 (d, = 2, 1H : H en 10) ; 5,59 (d, J = 7, 1H : H en 2) ; 7,45 (t, J = 7,5, 2H : OCOC₆H₅ H en méta) ; 7,57 (t, J = 7,5, 1H : OCOC₆H₅ H en para) ; 8,07 (d, J = 7,5, 2H : OCOC₆H₅ H en ortho).

Le carbonate-1β,2α ditriéthylsilyloxy-7β,13α époxy-5β,20 méthoxy-4α méthoxyacétoxy-10β oxo-9 taxène-11 peut être préparé de la manière suivante :

A une solution de 510 mg de carbonate-1β,2α ditriéthylsilyloxy-7β,13α époxy-5β,20 hydroxy-4α méthoxyacétoxy-10β oxo-9 taxène-11 dans 6 cm3 de diméthylformamide, on ajoute 0,3 g de tamis moléculaire 4A, 12 cm3 de iodure de méthyle et 90 mg d'hydrure de sodium, à une température voisine de 20°C et sous atmosphère d'argon. Le milieu réactionnel est agité à une température voisine de 20°C pendant 3 heures. On ajoute 10 cm3 d'une solution aqueuse saturée de chlorure d'ammonium et 30 cm3 de dichlorométhane. La phase organique est séparée par décantation, lavée par 2 fois 10 cm3 d'une solution aqueuse saturée de chlorure de sodium puis séchée sur sulfate de magnésium. Après filtration et concentration à sec sous pression réduite (2,7 kPa) à 40°C, on obtient 715 mg d'un produit que l'on purifie par chromatographie sur 50 g de silice (0,063-0,2 mm) contenus dans une colonne de 1 cm de diamètre en éluant avec un mélange acétate d'éthyle-cyclohexane (25-75 en volumes) en recueillant des fractions de 10 cm3. Les fractions ne contenant que le produit cherché sont réunies et concentrées à sec sous pression réduite (2,7 kPa) à 40°C. On obtient ainsi 494 mg de carbonate-1β,2α ditriéthylsilyloxy-7β,13α époxy-5β,20 méthoxy-4α méthoxyacétoxy-10β oxo-9 taxène-11, sous forme d'une meringue blanche, dont les caractéristiques physiques sont les suivantes :
- spectre de R.M.N. du proton (400 MHz ; CDCl₃ ; δ en ppm ; constantes de couplage J en Hz) : 0,60 (q, J = 7,5, 6 H : CH₂ éthyle) ; 0,68 (q, J = 7,5, 6 H : CH₂ éthyle) ; 0,92 (t, J = 7,5, 9H : CH₃ éthyle) ; 1,03 (t, J = 7,5, 9H : CH₃ éthyle) ; 1,19 (s, 3H : CH₃) ; 1,23 (s, 3H : CH₃) ; 1,44 (s, 1H : OH en 1) ; 1,71 (s, 3H : CH₃) ; 1,99 et 2,47 (2 mts, 1H chacun : CH₂ en 6) ; 2,15 (s, 3H : CH₃) ; 2,32 et 2,93 (2 dd, J = 15 et 9 et J = 15 et 6,5, 1H chacun : CH₂ en 14) ; 2,89 (d, J = 5, 1H : H en 3) ; 3,45 et 3,51 (2 s, 3H chacun : OCH₃) ; 4,10 (dd, J = 10,5 et 7, 1H : H en 7) ; 4,17 (AB limite, J = 16, 2H : OCOCH₂O) ; 4,41 (d, J = 5, 1H : H en 2) ; 4,43 et 4,79 (2 d, J = 10, 1H chacun : CH₂ en 20) ; 4,93 (mt, 1H : H en 13) ; 5,10 (d large, J = 10, 1H : H en 5) ; 6,51 (s, 1H : H en 10).

### EXEMPLE 2

A une solution de 18 mg de tert-butoxycarbonylamino-3 hydroxy-2 phényl-3 propionate-(2R,3S) de benzoyloxy-2α dihydroxy-1β,10β époxy-5β,20 éthoxy-4α oxo-9 tifluorométhanesulfonate-7β taxène-11 yle-13α dans 233 µl d'acétonitrile et 23 µl de tétrahydrofurane, on ajoute successivement 15 mg de tamis moléculaire 4Å en poudre et 27 mg de chlorure de sodium. Le milieu réactionnel est maintenu sous agitation à une température voisine de 75°C pendant 3 heures puis, à une température voisine de 20°C, additionné de 15 cm3 de dichlorométhane et 15 cm3 d'une solution aqueuse saturée de chlorure de sodium. La phase organique est décantée, lavée par 2 fois 10 cm3 d'une solution aqueuse saturée de chlorure de sodium puis séchée sur sulfate de magnésium, filtrée et concentrée à sec sous pression réduite (2,7 kPa) à 40°C. On obtient 22 mg d'un produit que l'on purifie par chromatographie préparative sur plaque de silice de 0,25 mm d'épaisseur en éluant avec un mélange dichlorométhane-méthanol (95-5 en volumes). On obtient ainsi 10 mg de tert-butoxycarbonylamino-3 hydroxy-2 phényl-3 propionate-(2R,3S) de benzoyloxy-2α dihydroxy-1β,10β époxy-5β,20 éthoxy-4α méthylène-7β,8 nor-19 oxo-9 taxène-11 yle-13α sous forme d'une meringue blanche et dont les caractéristiques physiques sont les suivantes :
- spectre de R.M.N. du proton (400 Mhz ; CDCl₃ ; δ en ppm ; constantes de couplage J en Hz) : 1,21 (s, 3H : CH₃) ; 1,28 (s, 3H : CH₃) ; 1,37 (s, 9H : C(CH₃)₃) ; 1,35 (mt, 1H : H 7) ; 1,47 (t, J = 7, 3H : CH₃ de C₂H₅ en 4) ; 1,72 (s, 1H : OH en 1) ; 1,84 et 2,32 (respectivement t et dd, J = 6 et J = 10 et 6, 1H chacun : CH₂ en 19) ; 1,89 (s, 3H : CH₃) ; 2,03 et 2,22 (respectivement dt et d large, J = 16 et 4 et J = 16 , 1H chacun : CH₂ en 6) ; 2,20 et 2,90 (respectivement dd et dd large, J = 16 et 9, 1H chacun : CH₂ en 14); 3,22 (mf, 1H : OH en 2') ; 3,47 et 3,68 (2 mts, 1H chacun : CH₂ de C₂H₅ en 4) ; 3,65 (d, J = 7, 1H : H en 3) ; 4,02 et 4,39 (2 d, J = 9, 1H chacun : CH₂ en 20) ; 4,26 (s large, 1H : OH en 10); 4,61 (mt, 1H : H en 2') ; 4,87 (mt, 1H : H en 5) ; 4,95 (s large, 1H : H en 10) ; 5,33 (d large, J = 10, 1H : H en 3') ; 5,42 (d, J = 10, 1H : CONH) ; 5,67 (d, J = 7, 1H : H en 2) ; 6,28 (t large, = 9, 1H : H en 13) ; de 7,30 à 7,45 (mt, 5H : H aromatiques en 3') ; 7,49 (t, J = 7,5, 2H : OCOC₆H₅ H en méta) ; 7,60 (t, J = 7,5, 1H : OCOC₆H₅ H en para) ; 8,11 (d, J = 7,5, 2H : OCOC₆H₅ H en ortho).

Le tert-butoxycarbonylamino-3 hydroxy-2 phényl-3 propionate-(2R,3S) de benzoyloxy-2α dihydroxy-1β,10β époxy-5β,20 éthoxy-4α oxo-9 trifluorométhanesulfonyloxy-7β taxène-11 yle-13α peut être préparé de la manière suivante :

Une solution de 66 mg de tert-butoxycarbonyl-3 (méthoxy-4 phényl)-2 phényl-4 oxazolidine-1,3 carboxylate-5-(2R,4S,5R) de benzoyloxy-2α dihydroxy1β,10β époxy-5β,20 éthoxy-4α oxo-9 trifluorométhanesulfonyloxy-7β taxène-11 yle-13α dans 1,5 ml d'une solution 0,1N d'éthanol chlorhydrique est maintenue sous agitation à une température voisine de 0°C pendant 19 heures. Le mélange réactionnel est concentré à sec sous pression réduite (2,7 kPa) à 20°C. On obtient 82 mg d'un produit que l'on purifie par chromatographie préparative sur plaque de silice de 0,25 mm d'épaisseur en éluant avec un mélange dichlorométhane-méthanol (95-5 en volumes). On obtient ainsi 20 mg de tert-butoxycarbonylamino-3 hydroxy-2 phényl-3 propionate-(2R,3S) de benzoyloxy-2α dihydroxy-1β,10β époxy-5β,20 éthoxy-4α oxo-9 trifluorométhanesulfonyloxy-7β taxène-11 yle-13α sous forme d'une meringue blanche et dont les caractéristiques physiques sont les suivantes :
- spectre de R.M.N. du proton (600 MHz ; CDCl₃ ; δ en ppm ; constantes de couplage J en Hz): 1,13 (s, 3H: CH₃); 1,25 (s, 3H : CH₃) ; 1,40 (s, 9H : C(CH₃)₃) ; 1,47 (t, J = 7, 3H : CH₃ de C₂H₅ en 4) ; 1,58 (s, 1H : OH en 1) ; 1,90 et 2,25 (respectivement mt et dd, J = 16 et 9, 1H chacun : CH₂ en 14) ; 1,92 (s, 3H : CH₃) ; 1,94 (s, 3H : CH₃) ; 2,40 et 2,70 (2 mts, 1H chacun : CH₂ en 6) ; 3,18 (s large, 1H : OH en 2') ; 3,43 (d, J = 6,5, 1H : H en 3) ; 3,75 et 3,82 (2 mts, 1H chacun : CH₂ de C₂H₅ en 4) ; 4,05 (s large, 1H : OH en 10) ; 4,28 et 4,46 (2 d, J = 9, 1H chacun : CH₂ en 20) ; 4,63 (mt, 1H : H en 2') ; 4,92 (dd, J = 11 et 7, 1H : H en 7) ; 5,03 (dd, J = 10 et 2, 1H : H en 5) ; 5,32 (mt, 1H : H en 3') ;5,33 (s large, 1H : H en 10) ; 5,45 (d, J = 10, 1H : CONH) ; 5,65 (d, J = 6,5, 1H : H en 2) ; 6,20 (t large, = 9, 1H : H en 13) ; de 7,30 à 7,55 (mt, 5H : H aromatiques en 3') ; 7,49 (t, J= 7,5, 2H : OCOC₆H₅ H en méta) ; 7,61 (t, J = 7,5, 1H : OCOC₆H₅ H en para) ; 8,02 (d, J = 7,5, 2H : OCOC₆H₅ H en ortho).

Le tert-butoxycarbonyl-3 (méthoxy-4 phényl)-2 phényl-4 oxazolidine-1,3 carboxylate-5 (2R,4S,5R) de benzoyloxy-2α dihydroxy-1β,10β époxy-5β,20 éthoxy-4α oxo-9 trifluorométhanesulfonate-7β taxène-11 yle-13α peut être préparé de la manière suivante :

A une solution de 90 mg de benzoyloxy-2α époxy-5β,20 éthoxy-4α oxo-9 trifluorométhanesulfonyloxy-7β trihydroxy-1β,10β,13α taxène-11 dans 4 cm3 d'acétate d'éthyle anhydre, on ajoute successivement 60 mg d'acide tert-butoxycarbonyl-3 (méthoxy-4 phényl)-2 phényl-4 oxazolidine-1,3 carboxylique-5-(2R,4S,5R), 42 mg de dicyclohexylcarbodiimide et 5 mg de 4-diméthylaminopyridine à une température voisine de 20°C. Le mélange réactionnel est agité pendant 6 heures, sous atmosphère d'argon, à une température voisine de 20°C. On ajoute 30 cm3 d'acétate d'éthyle et 20 cm3 d'une solution aqueuse saturée de chlorure d'ammonium. La phase organique est décantée, lavée par 2 fois 20 cm3 d'eau puis séchée sur sulfate de magnésium, filtrée et concentrée à sec sous pression réduite (2,7 kPa) à 40°C. On obtient 140 mg d'un produit que l'on purifie par chromatographie sur 30 g de silice (0,063-0,2 mm) contenus dans une colonne de 1 cm de diamètre en éluant avec un mélange cyclohexane-acétate d'éthyle (70-30 en volumes) en recueillant des fractions de 8 cm3. Les fractions ne contenant que le produit cherché sont réunies et concentrées à sec sous pression réduite (2,7 kPa) à 40°C. On obtient ainsi 110 mg de tert-butoxycarbonyl-3 (méthoxy-4 phényl)-2 phényl-4 oxazolidine-1,3 carboxylate-5 (2R,4S,5R) de benzoyloxy-2α dihydroxy-1β,10β époxy-5β,20 éthoxy-4α oxo-9 trifluorométhanesulfonyloxy-7β taxène-11 yle-13α sous forme d'une meringue blanche et dont les caractéristiques physiques sont les suivantes :
- spectre de R.M.N. du proton (400 MHz ; CDCl₃ ; δ en ppm ; constantes de couplage J en Hz) : 1,10 (s, 15H : C(CH₃)₃ - CH₃ et CH₃ de C₂H₅ en 4) ; 1,19 (s, 3H : CH₃) ; 1,51 (s, 1H : OH en 1) ; 1,64 (s, 3H : CH₃) ; 1,85 (s, 3H : CH₃) ; de 2,25 à 2,40 et 2,66 (2 mts, 1H chacun : CH₂ en 6) ; de 2,25 à 2,40 et 2,88 (respectivement mt et dd, J = 16 et 8, 1H chacun : CH₂ en 14) ; 3,35 (d, J = 6,5, 1H : H en 3) ; 3,52 et 3,62 (2 mts, 1H chacun : CH₂ de C₂H₅ en 4) ; 3,84 (s, 3H : ArOCH₃) ; 4,01 (d, J = 1, 1H : OH en 10) ; 4,20 et 4,34 (2d, J = 9, 1H chacun : CH₂ en 20) ; 4,64 (d, J = 4, 1H : H en 2') ; 4,85 (dd, J = 11,5 et 6,5, 1H : H en 7) ; 4,92 (d large, J =10,5, 1H : H en 5) ; 5,26 (d, J =1, 1H : H en 10) ; 5,55 (mf étalé, 1H : H en 3') ; 5,59 (d, J = 6,5, 1H : H en 2) ; 5,91 (mt, 1H : H en 13) ; 6,40 (mf étalé, 1H : H en 5') ; 6,94 (d, J = 8,5, 2H : H en ortho du OCH₃) ; de 7,30 à 7,50 (mt, 9H : H aromatiques en 3', H en méta du OCH₃ et OCOC₆H₅ H en méta) ; 7,63 (t, J = 7,5, 1H : OCOC₆H₅ H en para) ; 7,95 (d, J = 7,5, 2H : OCOC₆H₅ H en ortho).

Le benzoyloxy-2α époxy-5β,20 éthoxy-4α oxo-9 trifluorométhanesulfonyloxy-7β trihydroxy-1β,10β,13α taxène-11 peut être préparé de la manière suivante :

A une solution de 260 mg de benzoyloxy-2α époxy-5β,20 éthoxy-4α oxo-9 tétrahydroxy-1β,7β,10β,13α taxène-11 dans 10 cm3 de dichlorométhane et 145 µl de pyridine, on ajoute 200 µl d'anhydride triflique à une température voisine de 0°C. le mélange réactionnel est agité pendant 45 minutes à une température voisine de 0°C puis on ajoute 15 cm3 de dichlorométhane et 10 cm3 d'eau. La phase organique est décantée, lavée par 2 fois 10 cm3 d'une solution aqueuse saturée d'hydrogénocarbonate de sodium puis séchée sur sulfate de magnésium, filtrée et concentrée à sec sous pression réduite (2,7 kPa) à 20°C. On obtient 308 mg d'un produit que l'on purifie par chromatographie sur 60 g de silice (0,063-0,2 mm) contenus dans une colonne de 1 cm de diamètre en éluant avec un mélange acétate d'éthyle-cyclohexane (40-60 en volumes) en recueillant des fractions de 10 cm3. Les fractions ne contenant que le produit cherché sont réunies et concentrées à sec sous pression réduite (2,7 kPa) à 40°C. On obtient ainsi 90 mg de benzoyloxy-2α époxy-5β,20 éthoxy-4α trihydroxy-1β,10β,13α oxo-9 trifluorométhanesulfonyloxy-7β taxène-11 sous forme d'une meringue blanche et dont les caractéristiques physiques sont les suivantes :
- spectre de R.M.N. du proton (300 MHz ; CDCl₃ ; δ en ppm ; constantes de couplage J en Hz) : 1,07 (s, 3H : CH₃) ; 1,12 (s, 3H : CH₃) ; 1,47 (t, J J = 7, 3H : CH₃ de C₂H₅ en 4) ; 1,87 (s, 3H : CH₃) ; 2,05 (s, 1H : OH en 1) ; 2,15 (s, 3H : CH₃) ; 2,38 et 2,75 (2 mts, 1H chacun : CH₂ en 6) ; 2,49 et 2,65 (2 dd, respectivement J = 16 et 9 et J = 16 et 3,5, 1H chacun : CH₂ en 14) ; 2,89 (d, J = 10, 1H : OH en 13) ; 3,72 (d, J = 6,5, 1H : H en 3) ; de 3,80 à 3,95 (mt, 2H : CH₂ de C₂H₅ en 4) ; 3,97 (d, J = 1, 1H : OH en 10) ; 4,30 et 4,48 (2d, J = 9, 1H chacun : CH₂ en 20) ; 4,57 (t large, = 10, 1H : H en 13) ; de 4,95 à 5,15 (mt, 2H : H 5 et H en 7) ; 5,42 (d, = 1, 1H : H en 10) ; 5,63 (d, J = 6,5, 1H : H en 2) ; 7,48 (t, J = 7,5, 2H : OCOC₆H₅ H en méta) ; 7,63 (t, J = 7,5, 1H : OCOC₆H₅ H en para) ; 8,00 (d, J = 7,5, 2H : OCOC₆H₅ H en ortho).

Le benzoyloxy-2α époxy-5β,20 éthoxy-4α oxo-9 tétrahydroxy-1β,7β,10β,13α taxène-11 peut être préparé de la manière suivante :

A une solution de 524 mg de benzoyloxy-2α époxy-5β,20 éthoxy-4α hydroxy-1β oxo-9 tris(triéthylsilyloxy)-7β,10β,13α taxène-11 dans 8 cm3 de dichlorométhane, on ajoute, à une température voisine de 20°C, 10 cm3 de complexe triéthylamine-acide fluorhydrique. Le mélange réactionnel est agité pendant 7 heures à une température voisine de 20°C puis on ajoute 100 cm3 de dichlorométhane et 200 cm3 d'une solution aqueuse saturée d'hydrogénocarbonate de sodium. La phase organique est décantée, lavée par 2 fois 50 cm3 d'une solution aqueuse saturée de chlorure de sodium puis séchée sur sulfate de magnésium, filtrée et concentrée à sec sous pression réduite (2,7 kPa) à 40°C. On obtient ainsi 260 mg de benzoyloxy-2α époxy-5β,20 éthoxy-4α oxo-9 tétrahydroxy-1β,7β,10β,13α taxène-11 sous forme d'une meringue blanche et dont les caractéristiques physiques sont les suivantes :
- spectre de R.M.N. du proton (400 MHz ; CDCl₃ ; δ en ppm ; constantes de couplage J en Hz) : 1,06 (s, 3H : CH₃) ; 1,12 (s, 3H : CH₃); 1,46 (t, J = 7, 3H : CH₃ de C₂H₅ en 4) ; 1,72 (s, 3H : CH₃) ; 1,99 et 2,50 (2 mts, 1H chacun : CH₂ en 6) ; 2,04 (s, 1H : OH en 1) ; 2,10 (s, 3H : CH₃) ; de 2,45 à 2,55 (mt, 1H : OH en 7); 2,50 et 2,65 (respectivement mt et dd, J = 16 et 3,5, 1H chacun : CH₂ en 14) ; 3,06 (d, = 11, 1H : OH en 13) ; 3,70 (d, J = 6,5, 1H : H en 3) ; 3,84 (mt, 1H : H en 7) ; 3,89 et 3,96 (2 mts, 1H chacun : CH₂ de C₂H₅ en 4) ; 4,15 (s large, 1H : OH en 10) ; 4,31 et 4,44 (2d, J = 9 Hz, 1H chacun : CH₂ en 20) ; 4,54 (t large, J = 10, 1H : H 13) ; 4,93 (dd, J = 10 et 3,5, 1H : H en 5) ; 5,28 (s, 1H : H en 10) ; 5,63 (d, J = 6,5, 1H : H 2) ; 7,48 (t, J = 7,5, 2H : OCOC₆H₅ H en méta) ; 7,61 (t, J = 7,5, 1H : OCOC₆H₅ H en para) ; 8,02 (d, J = 7,5, 2H : OCOC₆H₅ H en ortho).

Le benzoyloxy-2α époxy-5β,20 éthoxy-4α hydroxy-1β oxo-9 tris(triéthylsilyloxy)-7β,10β,13α taxène-11 peut être préparé selon l'une des méthodes suivantes :

1) A une solution de 253 mg de carbonate-1β,2α époxy-5β,20 éthoxy-4α oxo-9 tris(triéthylsilyloxy)-7β,10β,13α taxène-11 dans 13 cm3 de tétrahydrofurane anhydre, on ajoute 320 µl d'une solution 1M de phényllithium dans le tétrahydrofurane à une température voisine de -78°C. Le mélange réactionnel est agité pendant 1 heure 30 minutes à une température voisine de -78°C puis on ajoute 10 cm3 d'une solution aqueuse saturée de chlorure d'ammonium. A une température voisine de 20°C, on ajoute 10 cm3 d'eau et 50 cm3 de d'acétate d'éthyle. La phase organique est décantée, lavée par 2 fois 20 cm3 d'une solution aqueuse saturée de chlorure de sodium puis séchée sur sulfate de magnésium, filtrée et concentrée à sec sous pression réduite (2,7 kPa) à 40°C. On obtient 500 mg d'un produit que l'on purifie par chromatographie sur 50 g de silice (0,063-0,2 mm) contenus dans une colonne de 3 cm de diamètre en éluant avec un mélange acétate d'éthyle-cyclohexane (15-85 en volumes) en recueillant des fractions de 10 cm3. Les fractions ne contenant que le produit cherché sont réunies et concentrées à sec sous pression réduite (2,7 kPa) à 40°C. On obtient ainsi 260 mg de benzoyloxy-2α époxy-5β,20 éthoxy-4α hydroxy-1β oxo-9 tris(triéthylsilyloxy)-7β,10β,13α taxène-11 sous forme d'une meringue blanche et dont les caractéristiques physiques sont les suivantes :
- spectre de R.M.N. du proton (400 MHz ; CDCl₃ ; δ en ppm ; constantes de couplage J en Hz) : de 0,55 à 0,75 (mt, 18H : CH₂ de C₂H₅) ; de 0,90 à 1,10 (mt, 27H : CH₃ de C₂H₅) ; 1,15 (s, 3H : CH₃) ; 1,22 (s, 3H : CH₃); 1,38 (t, J = 7, 3H : CH₃ de C₂H₅ en 4) ; 1,50 (s, 1H : OH en 1) ; 1,65 (s, 3H : CH₃) ; 2,00 et 2,39 (2 mts, 1H chacun : CH₂ en 6) ; 2,02 (s, 3H : CH₃) ; 2,05 et 2,85 (2 dd, respectivement J = 16 et 9 et J = 16 et 8,5 , 1H chacun : CH₂ en 14) ; 3,43 (d, J = 6,5, 1H : H 3) ; 3,44 et 3,90 (2 mts, 1H chacun : CH₂ de C₂H₅ en 4) ; 3,91 (mt, 1H : H en 7) ; 4,20 et 4,30 (2d, J = 9, 1H chacun : CH₂ en 20) ; 4,93 (dd, J = 10 et 3,5, 1H : H en 5) ; 4,97 (t large, J = 9, 1H : H en 13) ; 5,17 (s, 1H : H en 10) ; 5,60 (d, J = 6,5, 1H : H 2) ; 7,45 (t, J = 7,5, 2H : OCOC₆H₅ H en méta) ; 7,57 (t, J = 7,5, 1H : OCOC₆H₅ H en para) ; 8,06 (d, J = 7,5, 2H : OCOC₆H₅ H en ortho).

Le carbonate-1β,2α époxy-5β,20 éthoxy-4α oxo-9 tris(triéthylsilyloxy)-7β,10β,13α taxène-11 peut être préparé de la manière suivante :

A une solution de 353 mg de carbonate-1β,2α époxy-5β,20 hydroxy-4α oxo-9 tris(triéthylsilyloxy)-7β,10β,13α taxène-11 dans 2,1 cm3 de diméthylformamide, on ajoute 0,3 g de tamis moléculaire 4Å, 4,2 cm3 de iodure d'éthyle et 68 mg d'hydrure de sodium à 80 %, à une température voisine de 20°C et sous atmosphère d'argon. Le mélange réactionnel est agité à une température voisine de 20°C pendant 1 heure. On ajoute 10 cm3 d'une solution aqueuse saturée de chlorure d'ammonium et 30 cm3 de dichlorométhane. La phase organique est décantée, lavée par 2 fois 10 cm3 d'une solution aqueuse saturée de chlorure de sodium puis séchée sur sulfate de magnésium, filtrée et concentrée à sec sous pression réduite (2,7 kPa) à 40°C. On obtient 500 mg d'un produit que l'on purifie par chromatographie sur 25 g de silice (0,063-0,2 mm) contenus dans une colonne de 1 cm de diamètre en éluant avec un mélange acétate d'éthyle-cyclohexane (10-90 en volumes) en recueillant des fractions de 10 cm3. Les fractions ne contenant que le produit cherché sont réunies et concentrées à sec sous pression réduite (2,7 kPa) à 40°C. On obtient ainsi 253 mg de carbonate-1β,2α époxy-5β,20 éthoxy-4α oxo-9 tris(triéthylsilyloxy)-7β,10β,13α taxène-11 sous forme d'une meringue blanche et dont les caractéristiques physiques sont les suivantes :
- spectre de R.M.N. du proton (400 MHz, ; CDCl₃ ; δ en ppm ; constantes de couplage J en Hz) : de 0,55 à 0,75 (mt, 18H : CH₂ de C₂H₅) ; de 0,90 à 1,10 (mt, 27H : CH₃ de C₂H₅) ; 1,17 (s, 3H : CH₃) ; 1,25 (s, 3H : CH₃) ; 1,25 (t, J = 7, 3H : CH₃ de C₂H₅ en 4) ; 1,68 (s, 3H : CH₃) ; 1,98 et 2,47 (2 mts, 1H chacun : CH₂ en 6) ; 1,98 (s, 3H : CH₃) ; 2,26 et 3,07 (2 dd, respectivement J = 16 et 9 et J = 16 et 7, 1H chacun : CH₂ en 14) ; 2,87 (d, J = 5 Hz, 1H : H en 3) ; 3,71 et 3,82 (2 mts, 1H chacun : CH₂ de C₂H₅ en 4) ; 4,05 (dd, J = 10 et 7, 1H : H en 7) ; 4,39 (d, J = 5, 1H : H en 2) ; 4,45 et 4,77 (2d, J = 9, 1H chacun : CH₂ en 20) ; 4,97 (mt, 1H H en 13) ; 5,03 (d large, J = 10, 1H : H en 5); 5,15 (s, 1H : H en 10).

Le carbonate-1β,2α époxy-5β,20 hydroxy-4α oxo-9 tris(triéthylsilyloxy)-7β,10β,13α taxène-11 peut être préparé de la manière suivante:

A une solution de 98 mg de bis(triéthylsilyloxy)-7β,13α carbonate-1β,2α dihydroxy-4α,10β époxy-5β,20 oxo-9 taxène-11 dans 1 cm3 de diméthylformamide, on ajoute 51 mg d'imidazole et 50 µL de chlorure de triéthylsilane à une température voisine de 20°C et sous atmosphère d'argon. Le mélange réactionnel est agité à une température voisine de 20°C pendant 72 heures. On ajoute 10 cm3 d'eau et 20 cm3 d'acétate d'éthyle. La phase organique est décantée, lavée par 2 fois 10 cm3 d'une solution aqueuse saturée de chlorure de sodium puis séchée sur sulfate de magnésium, filtrée et concentrée à sec sous pression réduite (2,7 kPa) à 40°C. On obtient 190 mg d'un produit que l'on purifie par chromatographie préparative sur couche mince de 2 mm d'épaisseur en éluant avec un mélange cyclohexane-acétate d'éthyle (75-25 en volumes). On obtient ainsi 58 mg de carbonate-1β,2α époxy-5β,20 hydroxy-4α oxo-9 tris(triéthylsilyloxy)-7β,10β,13α taxène-11 sous forme d'une meringue blanche et dont les caractéristiques physiques sont les suivantes :
- spectre de R.M.N. du proton (400 MHz ; CDCl₃ ; δ en ppm ; constantes de couplage J en Hz) : de 0,50 à 0,70 et 0,74 (2 mts, respectivement 12H et 6H : CH₂ de C₂H₅) ; de 0,90 à 1,10 (mt, 27H : CH₃ de C₂H₅) ; 1,14 (s, 3H : CH₃) ; 1,19 (s, 3H : CH₃) ; 1,63 (s, 3H : CH₃ ; 1,98 et 2,50 (2 mts, 1H chacun : CH₂ en 6) ; 1,98 (s, 3H : CH₃) ; 2,55 et 2,67 (2 dd, respectivement J = 16 et 9 et J = 16 et 3,5 , 1H chacun : CH₂ en 14) ; 3,00 (s, 1H : OH en 4) ; 3,11 (d, J = 5, 1H : H en 3) ; 4,14 (dd, J = 10 et 7, 1H : H en 7) ; 4,33 (d, J = 5, 1H : H 2) ; 4,54 (AB limite, J = 9, 2H : CH₂ en 20) ; 4,73 (d large, J = 9, 1H : H en 13) ; 4,77 (d large, J = 10, 1H : H en 5) ; 5,23 (s, 1H : H en 10).

Le bis(triéthylsilyloxy)-7β,13α carbonate-1β,2α dihydroxy-4α,10β époxy-5β,20 oxo-9 taxène-11 peut être préparé de la manière suivante :

A une solution de 108 mg de bis(triéthylsilyloxy)-7β,13α carbonate-1β,2α époxy-5β,20 hydroxy-4α méthoxyacétoxy-10β oxo-9 taxène-11 dans 3,5 cm3 de méthanol, on ajoute 0,3 g de tamis moléculaire 4Å et 470 mg de iodure de zinc, à une température voisine de 20°C et sous atmosphère d'argon. Le mélange réactionnel est agité à une température voisine de 20°C pendant 72 heures. On ajoute 10 cm3 d'eau et 20 cm3 d'acétate d'éthyle. La phase organique est décantée, lavée par 2 fois 10 cm3 d'une solution aqueuse saturée de chlorure de sodium puis séchée sur sulfate de magnésium, filtrée et concentrée à sec sous pression réduite (2,7 kPa) à 40°C. On obtient 90 mg d'un produit que l'on purifie par chromatographie préparative sur couche mince de 2 mm d'épaisseur en éluant avec un mélange cyclohexane-acétate d'éthyle (75-25 en volumes). On obtient ainsi 56 mg de bis(triéthylsilyloxy)-7β,13α carbonate-1β,2α dihydroxy-4α,10β époxy-5β,20 oxo-9 taxène-11 sous forme d'une meringue blanche dont les caractéristiques physiques sont les suivantes :
- spectre de R.M.N. du proton (400 MHz ; CDCl₃ ; δ en ppm ; constantes de couplage J en Hz) : 0,54 et 0,74 (2 mts, 6H chacun : CH₂ de C₂H₅) ; 0,91 et 1,03 (2 t, J = 7,5 Hz, 9H chacun : CH₃ de C₂H₅) ; 1,12 (s, 3H : CH₃) ; 1,20 (s, 3H : CH₃) ; 1,72 (s, 3H : CH₃) ; 1,98 et 2,46 (2 mts, 1H chacun : CH₂ en 6) ; 2,04 (s, 3H : CH₃) ; 2,55 et 2,67 (2 dd, respectivement J = 16 et 9 et J = 16 et 3,5, 1H chacun : CH₂ en 14) ; 3,00 (s, 1H : OH en 4) ; 3,14 (d, J = 5, 1H : H en 3) ; 4,07 (dd, J = 10 et 7, 1H : H en 7) ; 4,19 (d, J = 2, 1H : OH en 10) ; 4,33 (d, J = 5, 1H : H en 2) ; 4,54 (AB limite, J = 10, 2H : CH₂ en 20) ; 4,76 (d large, J = 9, 1H : H en 13) ; 4,82 (d large, J = 10, 1H : H en 5) ; 5,18 (d, = 2, 1H : H en 10).

2) A une solution de 200 mg de benzoyloxy-2α dihydroxy-1β,4α époxy-5β,20 oxo-9 tris(triéthylsilyloxy)-7β,10β,13α taxène-11 dans 6 cm3 de diméthylformamide, on ajoute 0,3 g de tamis moléculaire 4Å, 1 cm3 d'iodure d'éthyle et 34 mg d'hydrure de sodium à 50 %, à une température voisine de 20°C et sous atmosphère d'argon. Le mélange réactionnel est agité à une température voisine de 20°C pendant 1 heure. On ajoute 10 cm3 d'une solution aqueuse saturée de chlorure d'ammonium et 30 cm3 d'acétate d'éthyle. La phase organique est décantée, lavée par 2 fois 10 cm3 d'une solution aqueuse saturée de chlorure de sodium puis séchée sur sulfate de magnésium, filtrée et concentrée à sec sous pression réduite (2,7 kPa) à 40°C. On obtient 320 mg que l'on purifie par chromatographie sur 100 g de silice (0,063-0,2 mm) contenus dans une colonne de 1 cm de diamètre en éluant avec un mélange acétate d'éthyle-cyclohexane (10-90 en volume) en recueillant des fractions de 10 cm3. Les fractions ne contenant que le produit cherché sont réunies et concentrées à sec sous pression réduite (2,7 kPa) à 40°C. On obtient 38 mg de benzoyloxy-2α éthoxy-4α époxy-5β,20 hydroxy-1β oxo-9 tris(triéthylsilyloxy)-7β,10β,13α taxène-11 sous forme d'une meringue blanche et dont les caractéristiques physiques sont identiques au produit obtenu précédemment.

Le benzoyloxy-2α dihydroxy-1β,4α époxy-5β,20 oxo-9 tris(triéthylsilyloxy)-7β,10β,13α taxène-11 peut être préparé de la manière suivante :

A une solution de 4,2 g de benzoyloxy-2α époxy-5β,20 oxo-9 tétrahydroxy1β,4α,10β,13α triéthylsilyloxy-7β taxène-11, préparé selon D.G. Kingston et al. Tetrahedron Letters.35, 6839 (1994), dans 50 cm3 de diméthylformamide, on ajoute 4,6 g d'imidazole et 2,35 g de chlorure de triéthylsilane à une température voisine de 20°C et sous atmosphère d'argon. Le mélange réactionnel est agité à une température voisine de 20°C pendant 72 heures. On ajoute 30 cm3 d'eau et 100 cm3 d'acétate d'éthyle. La phase organique est décantée, lavée par 2 fois 30 cm3 d'une solution aqueuse saturée de chlorure de sodium puis séchée sur sulfate de magnésium, filtrée et concentrée à sec sous pression réduite (2,7 kPa) à 40°C. On obtient 12 g d'un produit que l'on purifie par chromatographie sur 100 g de silice (0,063-0,2 mm) contenus dans une colonne de 3 cm de diamètre en éluant avec un mélange acétate d'éthyle-cyclohexane (10-90 en volume) en recueillant des fractions de 20 cm3. Les fractions ne contenant que le produit cherché sont réunies et concentrées à sec sous pression réduite (2,7 kPa) à 40°C. On obtient ainsi 3,6 g de benzoyloxy-2α dihydroxy-1β,4α époxy-5β,20 oxo-9 tris(triéthylsilyloxy)-7β,10β,13α taxène-11 sous forme d'une meringue blanche et dont les caractéristiques physiques sont les suivantes :
- spectre de R.M.N. du proton (300 MHz ; CDCl₃ ; δ en ppm ; constantes de couplage J en Hz) : 0,57 et de 0,60 à 0,85 (2 mts, respectivement 6H et 12H : CH₂ de C₂H₅) ; de 0,90 à 1,10 (mt, 30H : CH₃ de C₂H₅ et CH₃) ; 1,21 (s, 3H : CH₃) ; 1,53 (s, 3H : CH₃) ; 1,63 (s, 1H : OH en 1) ; 1,96 (s, 3H : CH₃) ; 1,97 et 2,45 (2 mts, 1H chacun : CH₂ en 6) ; 2,32 et 2,60 (2 dd, respectivement J = 16 et 9 et J = 16 et 2,5, 1H chacun : CH₂ en 14) ; 3.61 (d, J = 6, 1H : H en 3) ; 3,80 (s large, 1H : OH en 4) ; 4,05 (dd, J = 11,5 et 6, 1H : H en 7) ; 4,23 et 4,27 (AB limite, J = 9, 2H : CH₂ en 20) ; 4,64 (d large, J = 9, 1H : H en 13) ; 4,71 (dd, J = 10 et 4, 1H : H en 5) ; 5,25 (s, 1H : H en 10) ; 5,54 (d, J = 6, 3H : H en 2) ; 7,42 (t, J = 7,5, 2H : OCOC₆H₅ H en méta) ; 7,55 (t, J = 7,5, 1H : OCOC₆H₅ H en para) ; 8,11 (d, J = 7,5, 2H : OCOC₆H₅ H en ortho).

Les nouveaux produits de formule générale (I) dans laquelle Z représente un radical de formule générale (II) manifestent une activité inhibitrice significative de la prolifération cellulaire anormale et possèdent des propriétés thérapeutiques permettant le traitement de malades ayant des conditions pathologiques associées à une prolifération cellulaire anormale. Les conditions pathologiques incluent la prolifération cellulaire anormale de cellules malignes ou non malignes de divers tissus et/ou organes, comprenant, de manière non limitative, les tissus musculaires, osseux ou conjonctifs, la peau, le cerveau, les poumons, les organes sexuels, les systèmes lymphatiques ou rénaux, les cellules mammaires ou sanguines, le foie, l'appareil digestif, le pancréas et les glandes thyroïdes ou adrénales. Ces conditions pathologiques peuvent inclure également le psoriasis, les tumeurs solides, les cancers de l'ovaire, du sein, du cerveau, de la prostate, du colon, de l'estomac, du rein ou des testicules, le sarcome de Kaposi, le cholangiocarcinome, le choriocarcinome, le neuroblastome, la tumeur de Wilms, la maladie de Hodgkin, les mélanomes, les myélomes multiples, les leucémies lymphocytaires chroniques, les lymphomes granulocytaires aigus ou chroniques. Les nouveaux produits selon l'invention sont particulièrement utiles pour le traitement du cancer de l'ovaire. Les produits selon l'invention peuvent être utilisés pour prévenir ou retarder l'apparition ou la réapparition des conditions pathologiques ou pour traiter ces conditions pathologiques.

Les produits selon l'invention peuvent être administrés à un malade selon différentes formes adaptées à la voie d'administration choisie qui, de préférence, est la voie parentérale. L'administration par voie parentérale comprend les administrations intraveineuse, intrapéritonéale, intramusculaire ou sous-cutanée. Plus particulièrement préférée est l'administration intrapéritonéale ou intraveineuse.

La présente invention comprend également les compositions pharmaceutiques qui contiennent au moins un produit de formule générale (I) en une quantité suffisante adaptée à l'emploi en thérapeutique humaine ou vétérinaire. Les compositions peuvent être préparées selon les méthodes habituelles en utilisant un ou plusieurs adjuvants, supports ou excipients pharmaceutiquement acceptables. Les supports convenables incluent les diluants, les milieux aqueux stériles et divers solvants non toxiques. De préférence les compositions se présentent sous forme de solutions ou de suspensions aqueuses, de solutions injectables qui peuvent contenir des agents émusifiants, des colorants, des préservatifs ou des stabilisants.

Le choix des adjuvants ou excipients peut être déterminé par la solubilité et les propriétés chimiques du produit, le mode particulier d'administration et les bonnes pratiques pharmaceutiques.

Pour l'administration parentérale, on utilise des solutions ou des suspensions stériles aqueuses ou non aqueuses. Pour la préparation de solutions ou de suspensions non aqueuses peuvent être utilisés des huiles végétales naturelles telle que l'huile d'olive, l'huile de sésame ou l'huile de paraffine ou les esters organiques injectables tel que l'oléate d'éthyle. Les solutions stériles aqueuses peuvent être constituées d'une solution d'un sel pharmaceutiquement acceptable en solution dans de l'eau. Les solutions aqueuses conviennent pour l'administration intraveineuse dans la mesure où le pH est convenablement ajusté et où l'isotonicité est réalisée, par exemple, par une quantité suffisante de chlorure de sodium ou de glucose. La stérélisation peut être réalisée par chauffage ou par tout autre moyen qui n'altère pas la composition.

Il est bien entendu que tous les produits entrant dans les compositions selon l'invention doivent être purs et non toxiques pour les quantités utilisées.

Les compositions peuvent contenir au moins 0,01 % de produit thérapeutiquement actif. La quantité de produit actif dans une composition est telle qu'une posologie convenable puisse être prescrite. De préférence, les compositions sont préparées de telle façon qu'une dose unitaire contienne de 0,01 à 1000 mg environ de produit actif pour l'administration par voie parentérale.

Le traitement thérapeutique peut être effectué concuremment avec d'autres traitements thérapeutiques incluant des médicaments antinéoplastiques, des anticorps monoclonaux, des thérapies immunologiques ou des radiothérapies ou des modificateurs des réponses biologiques. Les modificateurs des réponses incluent, de manière non limitative, les lymphokines et les cytokines telles que les interleukines, les interférons (α, β ou δ) et le TNF. D'autres agents chimiothérapeutiques utiles dans le traitement des désordres dus à la prolifération anormale des cellules incluent, de manière non limitative, les agents alkylants tels que les moutardes à l'azote comme la mechloretamine, le cyclophosphamide, le melphalan et le chlorambucil, des sulfonates d'alkyle comme le busulfan, les nitrosourées comme la carmustine, la lomustine, la sémustine et la streptozocine, les triazènes comme la dacarbazine, les antimétabolites comme les analogues de l'acide folique tel que le méthotrexate, les analogues de pyrimidine comme le fluorouracil et la cytambine, des analogues de purines comme la mercaptopurine et la thioguanine, des produits naturels tels que les alcaloïdes de vinca comme la vinblastine, la vincristine et la vendésine, des épipodophyllotoxines comme l'étoposide et le teniposide, des antibiotiques comme la dactinomycine, la daunorubicine, la doxorubicine, la bléomycine, la plicamycine et la mitomycine, des enzymes comme la L-asparaginase, des agents divers comme les complexes de coordination du platine tel que le cisplatine, les urées substituées tel que l'hydroxyurée, les dérivés de méthylhydrazine comme la procarbazine, les suppresseurs adrénocoticoïques comme le mitotane et l'aminoglutéthymide, les hormones et les antagonistes comme les adrénocorticostéroïdes comme la prednisone, les progestines comme le caproate d'hydroxyprogestérone, l'acétate de méthoxyprogestérone et l'acétate de megestrol, les oestrogènes comme le diéthylstilbestrol et l'éthynylestradiol, les antioestrogène comme le tamoxifène, les androgènes comme le propionate de testostérone et la fluoxymesterone.

Les doses utilisées pour mettre en oeuvre les méthodes selon l'invention sont celles qui permettent un traitement prophylactique ou un maximum de réponse thérapeutique. Les doses varient selon la forme d'administration, le produit particulier sélectionné et les caractéristiques propres du sujet à traiter. En général, les doses sont celles qui sont thérapeutiquement efficaces pour le traitement des désordres dus à une prolifération cellulaire anormale. Les produits selon l'invention peuvent être administrés aussi souvent que nécessaire pour obtenir l'effet thérapeutique désiré. Certains malades peuvent répondre rapidement à des doses relativement fortes ou faibles puis avoir besoin de doses d'entretien faibles ou nulles. Généralement, de faibles doses seront utilisées au début du traitement et, si nécessaire, des doses de plus en plus fortes seront administrées jusqu'à l'obtention d'un effet optimum. Pour d'autres malades il peut être nécessaire d'administrer des doses d'entretien 1 à 8 fois par jour, de préférence 1 à 4 fois, selon les besoins physiologiques du malade considéré. Il est aussi possible que pour certains malades il soit nécessaire de n'utiliser qu'une à deux administrations journalières.

Chez l'homme, les doses sont généralement comprises entre 0,01 et 200 mg/kg. Par voie intrapéritonéale, les doses seront en général comprises entre 0,1 et 100 mg/kg et, de préférence entre 0,5 et 50 mg/kg et, encore plus spécifiquement entre 1 et 10 mg/kg. Par voie intraveineuse, les doses sont généralement comprises entre 0,1 et 50 mg/kg et, de préférence entre 0,1 et 5 mg/kg et, encore plus spécifiquement entre 1 et 2 mg/kg. Il est entendu que, pour choisir le dosage le plus approprié, devront être pris en compte la voie d'administration, le poids du malade, son état de santé général, son âge et tous les facteurs qui peuvent influer sur l'efficacité du traitement.

L'exemple suivant illustre une composition selon l'invention.

### EXEMPLE

On dissout 40 mg du produit obtenu à l'exemple 1 dans 1 cm3 d'Emulphor EL 620 et 1 cm3 d'éthanol puis la solution est diluée par addition de 18 cm3 de sérum physiologique.

La composition est administrée par perfusion pendant 1 heure par introduction dans du soluté physiologique.

## Revendications

1. Nouveaux taxoïdes de formule générale : 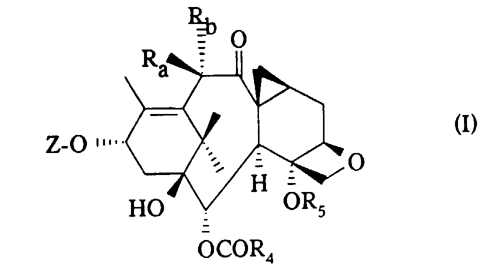 dans laquelle :
Rₐ représente un atome d'hydrogène ou un radical hydroxy, alcoxy contenant 1 à 4 atomes de carbone, acyloxy contenant 1 à 4 atomes de carbone ou alcoxyacétoxy dont la partie alcoyle contient 1 à 4 atomes de carbone et R_{b} représente un atome d'hydrogène ou bien Rₐ et R_{b} forment ensemble avec l'atome de carbone auquel ils sont liés une fonction cétone,
Z représente un atome d'hydrogène ou un radical de formule générale : 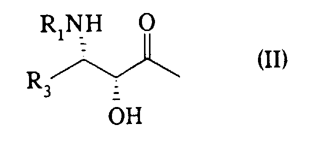 dans laquelle :
R₁ représente un radical benzoyle éventuellement substitué par un ou plusieurs atomes ou radicaux, identiques ou différents, choisis parmi les atomes d'halogène et les radicaux alcoyles contenant 1 à 4 atomes de carbone, alcoxy contenant 1 à 4 atomes de carbone ou trifluorométhyle, thénoyle ou furoyle, ou un radical R₂-O-CO- dans lequel R₂ représente :
- un radical alcoyle contenant 1 à 8 atomes de carbone, alcényle contenant 2 à 8 atomes de carbone, alcynyle contenant 3 à 8 atomes de carbone, cycloalcoyle contenant 3 à 6 atomes de carbone, cycloalcényle contenant 4 à 6 atomes de carbone, bicycloalcoyle contenant 7 à 10 atomes de carbone, ces radicaux étant éventuellement substitués par un ou plusieurs substituants choisis parmi les atomes d'halogène et les radicaux hydroxy, alcoxy contenant 1 à 4 atomes de carbone, dialcoylamino dont chaque partie alcoyle contient 1 à 4 atomes de carbone, pipéridino, morpholino, pipérazinyl-1 (éventuellement substitué en -4 par un radical alcoyle contenant 1 à 4 atomes de carbone ou par un radical phénylalcoyle dont la partie alcoyle contient 1 à 4 atomes de carbone), cycloalcoyle contenant 3 à 6 atomes de carbone, cycloalcényle contenant 4 à 6 atomes de carbone, phényle (éventuellement substitué par un ou plusieurs atomes ou radicaux choisis parmi les atomes d'halogène et les radicaux alcoyles contenant 1 à 4 atomes de carbone ou alcoxy contenant 1 à 4 atomes de carbone), cyano, carboxy ou alcoxycarbonyle dont la partie alcoyle contient 1 à 4 atomes de carbone,
- un radical phényle ou α- ou β-naphtyle éventuellement substitué par un ou plusieurs atomes ou radicaux choisis parmi les atomes d'halogène et les radicaux alcoyles contenant 1 à 4 atomes de carbone ou alcoxy contenant 1 à 4 atomes de carbone ou un radical hétérocyclique aromatique à 5 chaînons choisi de préférence parmi les radicaux furyle et thiényle,
- ou un radical hétérocyclyle saturé contenant 4 à 6 atomes de carbone éventuellement substitué par un ou plusieurs radicaux alcoyles contenant 1 à 4 atomes de carbone,
R₃ représente un radical alcoyle droit ou ramifié contenant 1 à 8 atomes de carbone, alcényle droit ou ramifié contenant 2 à 8 atomes de carbone, alcynyle droit ou ramifié contenant 2 à 8 atomes de carbone, cycloalcoyle contenant 3 à 6 atomes de carbone, phényle ou α- ou β-naphtyle éventuellement substitué par un ou plusieurs atomes ou radicaux choisis parmi les atomes d'halogène et les radicaux alcoyles, alcényles, alcynyles, aryles, aralcoyles, alcoxy, alcoylthio, aryloxy, arylthio, hydroxy, hydroxyalcoyle, mercapto, formyle, acyle, acylamino, aroylamino, alcoxycarbonylamino, amino, alcoylamino, dialcoylamino, carboxy, alcoxycarbonyle, carbamoyle, alcoylcarbamoyle, dialcoylcarbamoyle, cyano, nitro et trifluorométhyle, ou un hétérocycle aromatique ayant 5 chaînons et contenant un ou plusieurs hétéroatomes, identiques ou différents, choisis parmi les atomes d'azote, d'oxygène ou de soufre et éventuellement substitué par un ou plusieurs substituants, identiques ou différents, choisis parmi les atomes d'halogène et les radicaux alcoyles, aryles, amino, alcoylamino, dialcoylamino, alcoxycarbonylamino, acyle, arylcarbonyle, cyano, carboxy, carbamoyle, alcoylcarbamoyle, dialcoylcarbamoyle ou alcoxycarbonyle, étant entendu que, dans les substituants des radicaux phényle, α- ou β-naphtyle et hétérocyclyles aromatiques, les radicaux alcoyles et les portions alcoyles des autres radicaux contiennent 1 à 4 atomes de carbone et que les radicaux alcényles et alcynyles contiennent 2 à 8 atomes de carbone et que les radicaux aryles sont des radicaux phényles ou α- ou β-naphtyles, et
R₄ représente
- un radical alcoyle droit ou ramifié contenant 1 à 8 atomes de carbone, alcényle droit ou ramifié contenant 2 à 8 atomes de carbone, alcynyle droit ou ramifié contenant 2 à 8 atomes de carbone, cycloalcoyle contenant 3 à 6 atomes de carbone, cycloalcényle contenant 4 à 6 atomes de carbone ou bicycloalcoyle contenant 7 à 11 atomes de carbone, ces radicaux étant éventuellement substitués par un ou plusieurs substituants choisis parmi les atomes d'halogène et les radicaux hydroxy, alcoyloxy contenant 1 à 4 atomes de carbone, dialcoylamino dont chaque partie alcoyle contient 1 à 4 atomes de carbone, pipéridino, morpholino, pipérazinyl-1 (éventuellement substitué en -4 par un radical alcoyle contenant 1 à 4 atomes de carbone ou par un radical phénylalcoyle dont la partie alcoyle contient 1 à 4 atomes de carbone), cycloalcoyle contenant 3 à 6 atomes de carbone, cycloalcényle contenant 4 à 6 atomes de carbone, phényle éventuellement substitué, cyano, carboxy ou alcoyloxycarbonyle dont la partie alcoyle contient 1 à 4 atomes de carbone,
- ou un radical aryle éventuellement substitué par un ou plusieurs atomes ou radicaux choisis parmi les atomes d'halogène et les radicaux alcoyles, alcényles, alcynyles, aryles, aralcoyles, alcoxy, alcoylthio, aryloxy, arylthio, hydroxy, hydroxyalcoyle, mercapto, formyle, acyle, acylamino, aroylamino, alcoxycarbonyl-amino, amino, alcoylamino, dialcoylamino, carboxy, alcoxycarbonyle, carbamoyle, alcoylcarbamoyle, dialcoylcarbamoyle, cyano, nitro, azido, trifluorométhyle ou trifluorométhoxy, ou un radical hétérocyclyle saturé ou non saturé contenant 4 à 6 chaînons et éventuellement substitué par un ou plusieurs radicaux alcoyles contenant 1 à 4 atomes de carbone, et
R₅ représente un radical alcoyle droit ou ramifié contenant 1 à 8 atomes de carbone, alcényle droit ou ramifié contenant 2 à 8 atomes de carbone, alcynyle droit ou ramifié contenant 2 à 8 atomes de carbone, cycloalcoyle contenant 3 à 6 atomes de carbone, cycloalcényle contenant 4 à 6 atomes de carbone ou bicycloalcoyle contenant 7 à 11 atomes de carbone, ces radicaux étant éventuellement substitués par un ou plusieurs substituants choisis parmi les atomes d'halogène et les radicaux hydroxy, alcoyloxy contenant 1 à 4 atomes de carbone, dialcoylamino dont chaque partie alcoyle contient 1 à 4 atomes de carbone, pipéridino, morpholino, pipérazinyl-1 (éventuellement substitué en -4 par un radical alcoyle contenant 1 à 4 atomes de carbone ou par un radical phénylalcoyle dont la partie alcoyle contient 1 à 4 atomes de carbone), cycloalcoyle contenant 3 à 6 atomes de carbone, cycloalcényle contenant 4 à 6 atomes de carbone, phényle éventuellement substitué, cyano, carboxy ou alcoyloxycarbonyle dont la partie alcoyle contient 1 à 4 atomes de carbone,
étant entendu que les radicaux cycloalcoyles, cycloalcényles ou bicycloalcoyles peuvent être éventuellement substitués par un ou plusieurs radicaux alcoyles contenant 1 à 4 atomes de carbone.

2. Nouveaux taxoïdes selon la revendication 1 pour lesquels Rₐ représente un radical hydroxy, alcoxy contenant 1 à 4 atomes de carbone, acyloxy contenant 1 à 4 atomes de carbone ou alcoxyacétoxy dont la partie alcoyle contient 1 à 4 atomes de carbone et R_{b} représente un atome d'hydrogène, Z représente un atome d'hydrogène ou un radical de formule générale (II) dans laquelle R₁ représente un radical benzoyle ou un radical R₂-O-CO- dans lequel R₂ représente un radical tert-butyle et R₃ représente un radical alcoyle contenant 1 à 6 atomes de carbone, alcényle contenant 2 à 6 atomes de carbone, cycloalcoyle contenant 3 à 6 atomes de carbone, phényle éventuellement substitué par un ou plusieurs atomes ou radicaux, identiques ou différents choisis parmi les atomes d'halogène (fluor, chlore) et les radicaux alcoyles, alcoxy, dialcoylamino, acylamino, alcoxycarbonylamino ou trifluorométhyle ou un radical furyle-2 ou -3, thiényle-2 ou -3 ou thiazolyle-2, -4 ou -5 et R₄ représente un radical phényle éventuellement substitué par un ou plusieurs atomes ou radicaux, identiques ou différents, choisis parmi les atomes d'halogène et les radicaux alcoyles, alcoxy, amino, alcoylamino, dialcoylamino, acylamino, alcoxycarbonylamino, azido, trifluorométhyle et trifluorométhoxy, ou un radical thiényle-2 ou -3 ou furyle-2 ou -3 et R₅ représente un radical alcoyle éventuellement substitué contenant 1 à 4 atomes de carbone.

3. Nouveaux taxoïdes selon la revendication 1 pour lesquels Rₐ représente un atome d'hydrogène ou un radical hydroxy ou acétyloxy ou méthoxyacétoxy et R_{b} représente un atome d'hydrogène, Z représente un atome d'hydrogène ou un radical de formule générale (II) dans laquelle R₁ représente un radical benzoyle ou un radical R₂-O-CO- dans lequel R₂ représente un radical tert-butyle et R₃ représente un radical isobutyle, isobutényle, butényle, cyclohexyle, phényle, furyle-2, furyle-3, thiényle-2, thiényle-3, thiazolyle-2, thiazolyle-4 ou thiazolyle-5 et R₄ représente un radical phényle éventuellement substitué par un atome d'halogène et R₅ représente un radical alcoyle contenant 1 à 4 atomes de carbone.

4. Procédé de préparation d'un produit selon l'une des revendications 1,2 ou 3 caractérisé en ce que l'on traite par un halogénure de métal alcalin ou un azoture de métal alcalin ou un sel d'ammonium quaternaire ou un phosphate de métal alcalin un produit de formule générale : 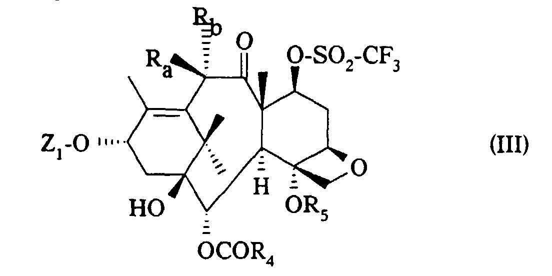 dans laquelle Z₁ représente un atome d'hydrogène ou un radical de formule générale (II) dans laquelle R₁ et R₃ sont définis comme précedemment ou un radical de formule générale : 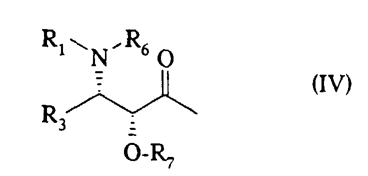 dans laquelle R₁ et R₃ sont définis comme précedemment, et, ou bien R₆ représente un atome d'hydrogène et R₇ représente un groupement protecteur de la fonction hydroxy, ou bien, R₆ et R₇ forment ensemble un hétérocycle saturé à 5 ou 6 chaînons, R₄ et R₅ sont définis comme précédemment, Rₐ représente un atome d'hydrogène ou un radical alcoxy, acyloxy, alcoxyacétoxy ou un radical hydroxy protégé, de préférence un radical trichloro-2,2,2 éthoxycarbonyloxy, et R_{b} représente un atome d'hydrogène, ou bien Rₐ et R_{b} forment ensemble avec l'atome d'azote auquel ils sont liés une fonction cétone, pour obtenir un produit de formule générale : 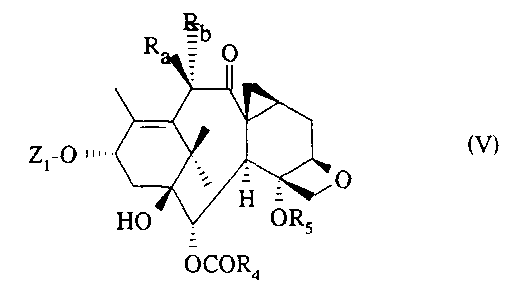 dans laquelle Z₁, R₄, R₅, Rₐ et R_{b} sont définis comme précédemment, suivi, si nécessaire, du remplacement du groupement protecteur porté par Rₐ ou des groupements protecteurs représentés par R₇ et/ou par R₆ et R₇ par des atomes d'hydrogène.

5. Procédé de préparation d'un produit selon l'une des revendications 1 à 3 pour lequel R₄ et R₅ sont définis comme dans l'une des revendications 1 à 3 et Rₐ et R_{b} représentent chacun un atome d'hydrogène caractérisé en ce que l'on réduit par voie électrolytique un produit selon l'une des revendications 1 à 3 pour lequel Rₐ représente un radical hydroxy, acyloxy ou alcoxyacétoxy.

6. Procédé de préparation d'un produit selon l'une des revendications 1 à 3 pour lequel R₄ et R₅ sont définis comme dans l'une des revendications 1 à 3, Rₐ et R_{b} forment ensemble avec l'atome de carbone auquel ils sont liés une fonction cétone caractérisé en ce que l'on oxyde un produit selon l'une des revendications 1 à 3 pour lequel Rₐ représente un radical hydroxy et R_{b} représente un atome d'hydrogène.

7. Composition pharmaceutique caractérisée en ce qu'elle contient au moins un produit selon l'une des revendications 1 à 3 pour lequel Z représente un radical de formule générale (II) en association avec un ou plusieurs produits pharmaceutiquement acceptables qu'ils soient inertes ou pharmacologiquement actifs.

## Patentansprüche

1. Neue Taxoide der allgemeinen Formel (I) 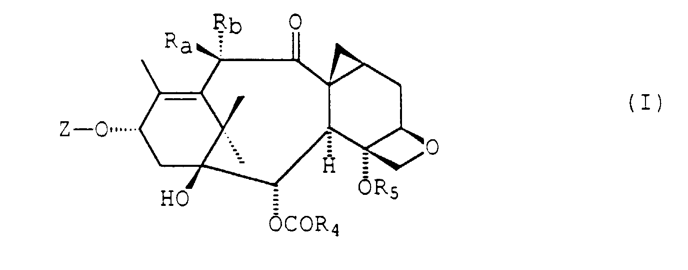 in der
Rₐ ein Wasserstoffatom oder einen Rest Hydroxy, Alkoxy mit 1 bis 4 Kohlenstoffatomen, Acyloxy mit 1 bis 4 Kohlenstoffatomen oder Alkoxyacetoxy darstellt, dessen Teil Alkyl 1 bis 4 Kohlenstoffatome enthält und R_{b} ein Wasserstoffatom ist, oder Rₐ und R_{b} zusammen mit dem Kohlenstoffatom, an das sie gebunden sind, eine Ketonfunktion bilden,
Z ein Wasserstoffatom oder einen Rest der allgemeinen Formel (II) 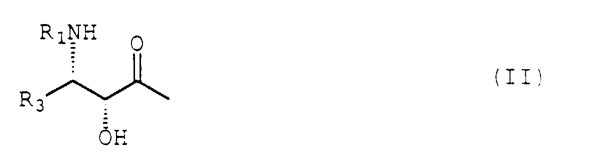 darstellt, in der
R₁ einen Rest Benzoyl darstellt, gegebenenfalls substituiert durch ein oder mehrere gleiche oder verschiedene Atome oder Reste, ausgewählt unter den Halogenatomen und den Resten Alkyl mit 1 bis 4 Kohlenstoffatomen, Alkoxy mit 1 bis 4 Kohlenstoffatomen oder Trifluormethyl, Thenoyl oder Furoyl, oder einen Rest R₂-O-CO-bedeutet, worin R₂ ist:
- ein Rest Alkyl mit 1 bis 8 Kohlenstoffatomen, Alkenyl mit 2 bis 8 Kohlenstoffatomen, Alkinyl mit 3 bis 8 Kohlenstoffatomen, Cycloalkyl mit 3 bis 6 Kohlenstoffatomen, Cycloalkenyl mit 4 bis 6 Kohlenstoffatomen, Bicycloalkyl mit 7 bis 10 Kohlenstoffatomen, wobei diese Reste gegebenenfalls substituiert sind durch einen oder mehrere Substituenten, ausgewählt unter den Halogenatomen und den Resten Hydroxy, Alkoxy mit 1 bis 4 Kohlenstoffatomen, Dialkylamino, bei dem jeder Teil Alkyl 1 bis 4 Kohlenstoffatome enthält, Piperidino, Morpholino, 1-Piperazinyl (gegebenenfalls substituiert in -4 durch einen Rest Alkyl mit 1 bis 4 Kohlenstoffatomen oder durch einen Rest Phenylalkyl, dessen Teil Alkyl 1 bis 4 Kohlenstoffatome enthält), Cycloalkyl mit 3 bis 6 Kohlenstoffatomen, Cycloalkenyl mit 4 bis 6 Kohlenstoffatomen, Phenyl (gegebenenfalls substituiert durch ein oder mehrere Atome oder Reste, ausgewählt unter den Halogenatomen und den Resten Alkyl mit 1 bis 4 Kohlenstoffatomen oder Alkoxy mit 1 bis 4 Kohlenstoffatomen), Cyano, Carboxy oder Alkoxycarbonyl, dessen Teil Alkyl 1 bis 4 Kohlenstoffatome enthält,
- ein Rest Phenyl oder α- oder β-Naphthyl, gegebenenfalls substituiert durch ein oder mehrere Atome oder Reste, ausgewählt unter den Halogenatomen und den Resten Alkyl mit 1 bis 4 Kohlenstoffatomen oder Alkoxy mit 1 bis 4 Kohlenstoffatomen, oder ein aromatischer heterocyclischer Rest mit 5 Ringgliedern, vorzugsweise ausgewählt unter den Resten Furyl und Thienyl, oder
- ein gesättigter heterocyclischer Rest mit 4 bis 6 Kohlenstoffatomen, gegebenenfalls substituiert durch einen oder mehrere Reste Alkyl mit 1 bis 4 Kohlenstoffatomen,
R₃ einen Rest Alkyl, gerade oder verzweigt und mit 1 bis 8 Kohlenstoffatomen, Alkenyl, gerade oder verzweigt und mit 2 bis 8 Kohlenstoffatomen, Alkinyl, gerade oder verzweigt und mit 2 bis 8 Kohlenstoffatomen, Cycloalkyl mit 3 bis 6 Kohlenstoffatomen, Phenyl oder α- oder β-Naphthyl darstellt, gegebenenfalls substituiert durch ein oder mehrere Atome oder Reste, ausgewählt unter den Halogenatomen und den Resten Alkyl, Alkenyl, Alkinyl, Aryl, Aralkyl, Alkoxy, Alkylthio, Aryloxy, Arylthio, Hydroxy, Hydroxyalkyl, Mercapto, Formyl, Acyl, Acylamino, Aroylamino, Alkoxycarbonylamino, Amino, Alkylamino, Dialkylamino, Carboxy, Alkoxycarbonyl, Carbamoyl, Alkylcarbamoyl, Dialkylcarbamoyl, Cyano, Nitro und Trifluormethyl, oder einen aromatischen Heterocyclus mit 5 Ringgliedern und einem oder mehreren, gleichen oder verschiedenen Heteroatomen, ausgewählt unter den Atomen von Stickstoff, Sauerstoff oder Schwefel, bedeutet, gegebenenfalls substituiert durch einen oder mehrere, gleiche oder verschiedene Substituenten, ausgewählt unter den Halogenatomen und den Resten Alkyl, Aryl, Amino, Alkylamino, Dialkylamino, Alkoxycarbonylamino, Acyl, Arylcarbonyl, Cyano, Carboxy, Carbamoyl, Alkylcarbamoyl, Dialkylcarbamoyl oder Alkoxycarbonyl, mit der Maßgabe, daß in den Substituenten der Reste Phenyl, α- oder β-Naphthyl und der aromatischen Heterocyclen die Reste Alkyl und die Teile Alkyl der anderen Reste 1 bis 4 Kohlenstoffatome enthalten, und daß die Reste Alkenyl und Alkinyl 2 bis 8 Kohlenstoffatome enthalten, und daß die Reste Aryl Reste Phenyl oder α- oder β-Naphthyl sind, und
R₄ einen Rest Alkyl, gerade oder verzweigt und mit 1 bis 8 Kohlenstoffatomen, Alkenyl, gerade oder verzweigt und mit 2 bis 8 Kohlenstoffatomen, Alkinyl, gerade oder verzweigt und mit 2 bis 8 Kohlenstoffatomen, Cycloalkyl mit 3 bis 6 Kohlenstoffatomen, Cycloalkenyl mit 4 bis 6 Kohlenstoffatomen oder Bicycloalkyl mit 7 bis 11 Kohlenstoffatomen darstellt, wobei diese Reste gegebenenfalls substituiert sind durch einen oder mehrere Substituenten, ausgewählt unter den Halogenatomen und den Resten Hydroxy, Alkyloxy mit 1 bis 4 Kohlenstoffatomen, Dialkylamino, bei dem jeder Teil Alkyl 1 bis 4 Kohlenstoffatomen enthält, Piperidino, Morpholino, 1-Piperazinyl (gegebenenfalls substituiert in -4 durch einen Rest Alkyl mit 1 bis 4 Kohlenstoffatomen oder durch einen Rest Phenylalkyl, dessen Teil Alkyl 1 bis 4 Kohlenstoffatome enthält), Cycloalkyl mit 3 bis 6 Kohlenstoffatomen, Cycloalkenyl mit 4 bis 6 Kohlenstoffatomen, Phenyl, gegebenenfalls substituiert, Cyano, Carboxy oder Alkyloxycarbonyl, dessen Teil Alkyl 1 bis 4 Kohlenstoffatome enthält,
- oder einen Rest Aryl darstellt, gegebenenfalls substituiert durch ein oder mehrere Atome oder Reste, ausgewählt unter den Halogenatomen und den Resten Alkyl, Alkenyl, Alkinyl, Aryl, Aralkyl, Alkoxy, Alkylthio, Aryloxy, Arylthio, Hydroxy, Hydroxyalkyl, Mercapto, Formyl, Acyl, Acylamino, Aroylamimo, Alkoxycarbonylamino, Amino, Alkylamino, Dialkylamino, Carboxy, Alkoxycarbonyl, Carbamoyl, Alkylcarbamoyl, Dialkylcarbamoyl, Cyano, Nitro, Azido, Trifluormethyl oder Trifluormethoxy,
oder einen gesättigten oder ungesättigten heterocyclischen Rest mit 4 bis 6 Ringgliedern bedeutet, gegebenenfalls substituiert durch ein oder mehrere Reste Alkyl mit 1 bis 4 Kohlenstoffatomen, und
R₅ einen Rest Alkyl, gerade oder verzweigt und mit 1 bis 8 Kohlenstoffatomen, Alkenyl, gerade oder verzweigt und mit 2 bis 8 Kohlenstoffatomen, Alkinyl, gerade oder verzweigt und mit 2 bis 8 Kohlenstoffatomen, Cycloalkyl mit 3 bis 6 Kohlenstoffatomen, Cycloalkenyl mit 4 bis 6 Kohlenstoffatomen oder Bicycloalkyl mit 7 bis 11 Kohlenstoffatomen darstellt, wobei diese Reste gegebenenfalls substituiert sind durch einen oder mehrere Substituenten, ausgewählt unter den Halogenatomen und den Resten Hydroxy, Alkyloxy mit 1 bis 4 Kohlenstoffatomen, Dialkylamino, bei dem jeder Teil Alkyl 1 bis 4 Kohlenstoffatomen enthält, Piperidino, Morpholino, 1-Piperazinyl (gegebenenfalls substituiert in -4 durch einen Rest Alkyl mit 1 bis 4 Kohlenstoffatomen oder durch einen Rest Phenylalkyl, dessen Teil Alkyl 1 bis 4 Kohlenstoffatome enthält), Cycloalkyl mit 3 bis 6 Kohlenstoffatomen, Cycloalkenyl mit 4 bis 6 Kohlenstoffatomen, Phenyl, gegebenenfalls substituiert, Cyano, Carboxy oder Alkyloxycarbonyl, dessen Teil Alkyl 1 bis 4 Kohlenstoffatome enthält,
mit der Maßgabe, daß die Reste Cycloalkyl, Cycloalkenyl oder Bicycloalkyl gegebenenfalls durch ein oder mehrere Reste Alkyl mit 1 bis 4 Kohlenstoffatomen substituiert sein können.

2. Neue Taxoide nach Anspruch 1, worin Rₐ einen Rest Hydroxy, Alkoxy mit 1 bis 4 Kohlenstoffatomen, Acyloxy mit 1 bis 4 Kohlenstoffatomen oder Alkoxyacetoxy darstellt, dessen Teil Alkyl 1 bis 4 Kohlenstoffatome enthält, und R_{b} ein Wasserstoffatom ist, Z ein Wasserstoffatom oder einen Rest der allgemeinen Formel (II) bedeutet, in der R₁ einen Rest Benzoyl oder einen Rest R₂-O-CO- darstellt, worin R₂ ein Rest tert.-Butyl ist und R₃ einen Rest Alkyl mit 1 bis 6 Kohlenstoffatomen, Alkenyl mit 2 bis 6 Kohlenstoffatomen, Cycloalkyl mit 3 bis 6 Kohlenstoffatomen, Phenyl, gegebenenfalls substituiert durch ein oder mehrere, gleiche oder verschiedene Atome oder Reste, ausgewählt unter den Halogenatomen (Fluor, Chlor) und den Resten Alkyl, Alkoxy, Dialkylamino, Acylamino, Alkoxycarbonylamino oder Trifluormethyl, oder einen Rest 2-Furyl oder 3-Furyl, 2-Thienyl oder 3-Thienyl oder 2-Thiazolyl, 4-Thiazolyl oder 5-Thiazolyl bedeutet, und R₄ einen Rest Phenyl, gegebenenfalls substituiert durch ein oder mehrere, gleiche oder verschiedene Atome oder Reste, ausgewählt unter den Halogenatomen und den Resten Alkyl, Alkoxy, Amino, Alkylamino, Dialkylamino, Acylamino, Alkoxycarbonylamino, Azido, Trifluormethyl und Trifluormethoxy, oder einen Rest 2-Thienyl oder 3-Thienyl oder 2-Furyl oder 3-Furyl darstellt, und R₅ ein gegebenenfalls substituierter Rest Alkyl mit 1 bis 4 Kohlenstoffatomen ist.

3. Neue Taxoide nach Anspruch 1, worin Rₐ ein Wasserstoffatom oder einen Rest Hydroxy oder Acetyloxy oder Methoxyacetoxy darstellt und R_{b} ein Wasserstoffatom ist, Z ein Wasserstoffatom oder einen Rest der allgemeinen Formel (II) bedeutet, in der R₁ einen Rest Benzoyl oder einen Rest R₂-O-CO- darstellt, worin R₂ ein Rest tert.-Butyl ist und R₃ einen Rest Isobutyl, Isobutenyl, Butenyl, Cyclohexyl, Phenyl, 2-Furyl, 3-Furyl, 2-Thienyl, 3-Thienyl, 2-Thiazolyl, 4-Thiazolyl oder 5-Thiazolyl bedeutet, und R₄ einen Rest Phenyl, gegebenenfalls substituiert durch ein Halogenatom, darstellt, und R₅ ein Rest Alkyl mit 1 bis 4 Kohlenstoffatomen ist.

4. Verfahren zur Herstellung eines Produktes nach einem der Ansprüche 1, 2 oder 3, dadurch gekennzeichnet, daß man mit einem Alkalimetallhalogenid oder einem Alkalimetallazid oder einem quaternären Ammoniumsalz oder einem Alkalimetallphosphat ein Produkt der allgemeinen Formel (III) 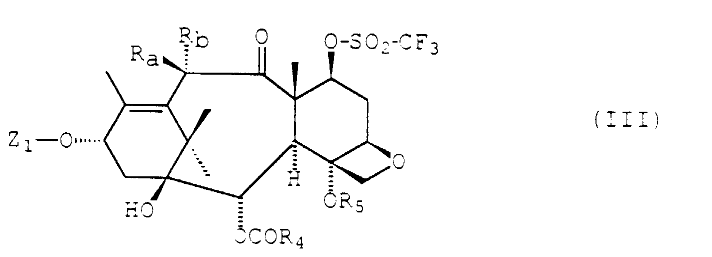 behandelt, in der Z₁ ein Wasserstoffatom oder ein Rest der allgemeinen Formel (II) ist, worin R₁ und R₃ wie vorstehend definiert sind oder einen Rest der allgemeinen Formel (IV) 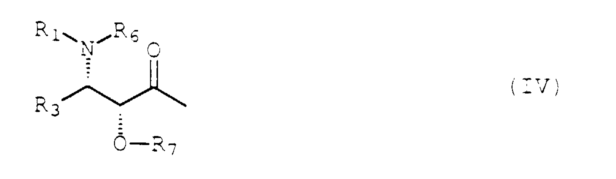 darstellen, in der R₁ und R₃ wie vorstehend definiert sind, und entweder R₆ ein Wasserstoffatom ist und R₇ eine Schutzgruppe für die Hydroxyfunktion darstellt, oder R₆ und R₇ zusammen einen gesättigten Heterocyclus mit 5 oder 6 Ringgliedern bilden, R₄ und R₅ wie vorstehend definiert sind, Rₐ ein Wasserstoffatom oder ein Rest Alkoxy, Acyloxy, Alkoxyacetoxy oder ein geschützter Rest Hydroxy ist, vorzugsweise ein Rest 2,2,2-Trichlor-ethoxycarbonyloxy, und R_{b} ein Wasserstoffatom bedeutet, oder Rₐ und R_{b} zusammen mit dem Stickstoffatom, an das sie gebunden sind, eine Ketonfunktion bilden, um ein Produkt der allgemeinen Formel (V) 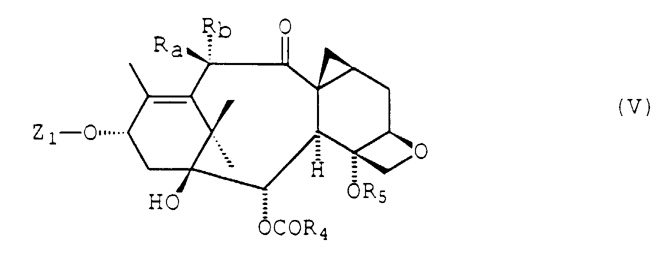 zu erhalten, in der Z₁, R₄, R₅, Rₐ und R_{b} wie vorstehend definiert sind, gefolgt, wenn erforderlich, von dem Austausch der von Rₐ getragenen Schutzgruppe oder der durch R₇ und/oder durch R₆ und R₇ dargestellten Schutzgruppen durch Wasserstoffatome.

5. Verfahren zur Herstellung eines Produktes nach einem der Ansprüche 1 bis 3, worin R₄ und R₅ wie in einem der Ansprüche 1 bis 3 definiert sind und Rₐ und R_{b} jeweils ein Wasserstoffatom darstellen, dadurch gekennzeichnet, daß man ein Produkt nach einem der Ansprüche 1 bis 3, worin Rₐ einen Rest Hydroxy, Acyloxy oder Alkoxyacetoxy bedeutet, auf elektrolytischem Wege reduziert.

6. Verfahren zur Herstellung eines Produktes nach einem der Ansprüche 1 bis 3, worin R₄ und R₅ wie in einem der Ansprüche 1 bis 3 definiert sind und Rₐ und R_{b} zusammen mit dem Stickstoffatom, an das sie gebunden sind, eine Ketonfunktion bilden, dadurch gekennzeichnet, daß man ein Produkt nach einem der Ansprüche 1 bis 3, worin Rₐ ein Rest Hydroxy ist und R_{b} ein Wasserstoffatom darstellt, oxidiert.

7. Pharmazeutische Zusammensetzungen, dadurch gekennzeichnet, daß sie mindestens ein Produkt nach einem der Ansprüche 1 bis 3, worin Z einen Rest der allgemeinen Formel (II) darstellt, in Assoziation mit einem oder mehreren pharmazeutisch akzeptablen Produkten, die inert oder pharmakologisch aktiv sind, enthalten.

## Claims

1. New taxoids of general formula: 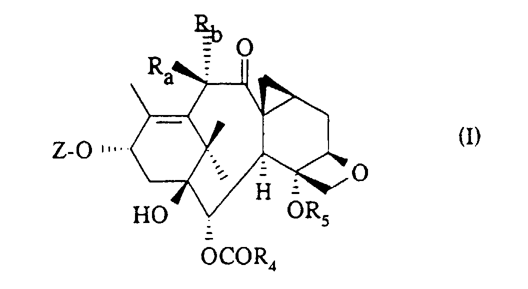 in which:
Rₐ represents a hydrogen atom or a hydroxyl radical, an alkoxy radical containing 1 to 4 carbon atoms, an acyloxy radical containing 1 to 4 carbon atoms or an alkoxyacetoxy radical in which the alkyl portion contains 1 to 4 carbon atoms and R_{b} represents a hydrogen atom, or alternatively Rₐ and R_{b}, together with the carbon atom to which they are attached, form a keto function,
Z represents a hydrogen atom or a radical of general formula: 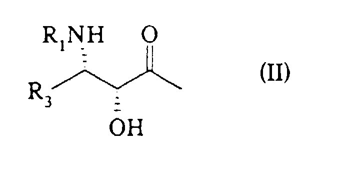 in which:
R₁ represents a benzoyl radical optionally substituted with one or more identical or different atoms or radicals chosen from halogen atoms and alkyl radicals containing 1 to 4 carbon atoms, alkoxy radicals containing 1 to 4 carbon atoms or trifluoromethyl radicals, a thenoyl or furoyl radical or a radical R₂-O-CO- in which R₂ represents:
- an alkyl radical containing 1 to 8 carbon atoms, an alkenyl radical containing 2 to 8 carbon atoms, an alkynyl radical containing 3 to 8 carbon atoms, a cycloalkyl radical containing 3 to 6 carbon atoms, a cycloalkenyl radical containing 4 to 6 carbon atoms or a bicycloalkyl radical containing 7 to 10 carbon atoms, these radicals being optionally substituted with one or more substituents chosen from halogen atoms and hydroxyl radicals, alkoxy radicals containing 1 to 4 carbon atoms, dialkylamino radicals in which each alkyl portion contains 1 to 4 carbon atoms, piperidino or morpholino radicals, 1-piperazinyl radicals (optionally substituted at position 4 with an alkyl radical containing 1 to 4 carbon atoms or with a phenylalkyl radical in which the alkyl portion contains 1 to 4 carbon atoms), cycloalkyl radicals containing 3 to 6 carbon atoms, cycloalkenyl radicals containing 4 to 6 carbon atoms, phenyl radicals (optionally substituted with one or more atoms or radicals chosen from halogen atoms and alkyl radicals containing 1 to 4 carbon atoms or alkoxy radicals containing 1 to 4 carbon atoms), cyano or carboxyl radicals or alkoxycarbonyl radicals in which the alkyl portion contains 1 to 4 carbon atoms,
- a phenyl or α- or β-naphthyl radical optionally substituted with one or more atoms or radicals chosen from halogen atoms and alkyl radicals containing 1 to 4 carbon atoms or alkoxy radicals containing 1 to 4 carbon atoms, or a 5-membered aromatic heterocyclic radical preferably chosen from furyl and thienyl radicals,
- or a saturated heterocyclic radical containing 4 to 6 carbon atoms, optionally substituted with one or more alkyl radicals containing 1 to 4 carbon atoms,
R₃ represents an unbranched or branched alkyl radical containing 1 to 8 carbon atoms, an unbranched or branched alkenyl radical containing 2 to 8 carbon atoms, an unbranched or branched alkynyl radical containing 2 to 8 carbon atoms, a cycloalkyl radical containing 3 to 6 carbon atoms, a phenyl or α- or β-naphthyl radical optionally substituted with one or more atoms or radicals chosen from halogen atoms and alkyl, alkenyl, alkynyl, aryl, aralkyl, alkoxy, alkylthio, aryloxy, arylthio, hydroxyl, hydroxyalkyl, mercapto, formyl, acyl, acylamino, aroylamino, alkoxycarbonylamino, amino, alkylamino, dialkylamino, carboxyl, alkoxycarbonyl, carbamoyl, alkylcarbamoyl, dialkylcarbamoyl, cyano, nitro and trifluoromethyl radicals, or a 5-membered aromatic heterocycle containing one or more identical or different hetero atoms chosen from nitrogen, oxygen and sulphur atoms and optionally substituted with one or more identical or different substituents chosen from halogen atoms and alkyl, aryl, amino, alkylamino, dialkylamino, alkoxycarbonylamino, acyl, arylcarbonyl, cyano, carboxyl, carbamoyl, alkylcarbamoyl, dialkylcarbamoyl or alkoxycarbonyl radicals, on the understanding that, in the substituents of the phenyl, α- or β-naphthyl and aromatic heterocyclic radicals, the alkyl radicals and the alkyl portions of the other radicals contain 1 to 4 carbon atoms, and that the alkenyl and alkynyl radicals contain 2 to 8 carbon atoms, and that the aryl radicals are phenyl or α- or β-naphthyl radicals, and
R₄ represents
- an unbranched or branched alkyl radical containing 1 to 8 carbon atoms, an unbranched or branched alkenyl radical containing 2 to 8 carbon atoms, an unbranched or branched alkynyl radical containing 2 to 8 carbon atoms, a cycloalkyl radical containing 3 to 6 carbon atoms, a cycloalkenyl radical containing 4 to 6 carbon atoms or a bicycloalkyl radical containing 7 to 11 carbon atoms, these radicals being optionally substituted with one or more substituents chosen from halogen atoms and hydroxyl radicals, alkyloxy radicals containing 1 to 4 carbon atoms, dialkyamino radicals in which each alkyl portion contains 1 to 4 carbon atoms, piperidino or morpholino radicals, 1-piperazinyl radicals (optionally substituted at position 4 with an alkyl radical containing 1 to 4 carbon atoms or with a phenylalkyl radical in which the alkyl portion contains 1 to 4 carbon atoms), cycloalkyl radicals containing 3 to 6 carbon atoms, cycloalkenyl radicals containing 4 to 6 carbon atoms, optionally substituted phenyl radicals, cyano or carboxyl radicals or alkyloxycarbonyl radicals in which the alkyl portion contains 1 to 4 carbon atoms,
- or an aryl radical optionally substituted with one or more atoms or radicals chosen from halogen atoms and alkyl, alkenyl, alkynyl, aryl, aralkyl, alkoxy, alkylthio, aryloxy, arylthio, hydroxyl, hydroxylalkyl, mercapto, formyl, acyl, acylamino, aroylamino, alkoxycarbonylamino, amino, alkylamino, dialkylamino, carboxyl, alkoxycarbonyl, carbamoyl, alkylcarbamoyl, dialkylcarbamoyl, cyano, nitro, azido, trifluoromethyl or trifluoromethoxy radicals,
or a saturated or unsaturated 4- to 6-membered heterocyclic radical optionally substituted with one or more alkyl radicals containing 1 to 4 carbon atoms, and
R⁵ represents an unbranched or branched alkyl radical containing 1 to 8 carbon atoms, an unbranched or branched alkenyl radical containing 2 to 8 carbon atoms, an unbranched or branched alkynyl radical containing 2 to 8 carbon atoms, a cycloalkyl radical containing 3 to 6 carbon atoms, a cycloalkenyl radical containing 4 to 6 carbon atoms or a bicycloalkyl radical containing 7 to 11 carbon atoms, these radicals being optionally substituted with one or more substituents chosen from halogen atoms and hydroxyl radicals, alkyloxy radicals containing 1 to 4 carbon atoms, dialkylamino radicals in which each alkyl portion contains 1 to 4 carbon atoms, piperidino or morpholino radicals, 1-piperazinyl radicals (optionally substituted at position 4 with an alkyl radical containing 1 to 4 carbon atoms or with a phenylalkyl radical in which the alkyl portion contains 1 to 4 carbon atoms), cycloalkyl radicals containing 3 to 6 carbon atoms, cycloalkenyl radicals containing 4 to 6 carbon atoms, optionally substituted phenyl radicals, cyano or carboxyl radicals or alkyloxycarbonyl radicals in which the alkyl portion contains 1 to 4 carbon atoms, on the understanding that the cycloalkyl, cycloalkenyl or bicycloalkyl radicals may be optionally substituted with one or more alkyl radicals containing 1 to 4 carbon atoms.

2. New taxoids according to claim 1 for which Rₐ represents a hydroxyl radical, an alkoxy radical containing 1 to 4 carbon atoms, an acyloxy radical containing 1 to 4 carbon atoms or an alkoxyacetoxy radical in which the alkyl portion contains 1 to 4 carbon atoms and R_{b} represents a hydrogen atom, Z represents a hydrogen atom or a radical of general formula (II) in which R₁ represents a benzoyl radical or a radical R₂-O-CO- in which R₂ represents a tert-butyl radical and R₃ represents an alkyl radical containing 1 to 6 carbon atoms, an alkenyl radical containing 2 to 6 carbon atoms, a cycloalkyl radical containing 3 to 6 carbon atoms, a phenyl radical optionally substituted with one or more identical or different atoms or radicals chosen from halogen atoms (fluorine, chlorine) and alkyl, alkoxy, dialkylamino, acylamino, alkoxycarbonylamino or trifluoromethyl radicals, or a 2- or 3-furyl, 2- or 3-thienyl or 2-, 4- or 5-thiazolyl radical, and R₄ represents a phenyl radical optionally substituted with one or more identical or different atoms or radicals chosen from halogen atoms and alkyl, alkoxy, amino, alkylamino, dialkylamino, acylamino, alkoxycarbonylamino, azido, trifluoromethyl and trifluoromethoxy radicals, or a 2- or 3-thienyl or 2- or 3-furyl radical, and R₅ represents an optionally substituted alkyl radical containing 1 to 4 carbon atoms.

3. New taxoids according to claim 1 for which Rₐ represents a hydrogen atom cr a hydroxyl or acetyloxy or methoxyacetoxy radical and R_{b} represents a hydrogen atom, Z represents a hydrogen atom or a radical of general formula (II) in which R₁ represents a benzoyl radical or a radical R₂-O-CO- in which R₂ represents a tert-butyl radical and R₃ represents an isobutyl, isobutenyl, butenyl, cyclohexyl, phenyl, 2-furyl, 3-furyl, 2-thienyl, 3-thienyl, 2-thiazolyl, 4-thiazolyl or 5-thiazolyl radical and R₄ represents a phenyl radical optionally substituted with a halogen atom and R₅ represents an alkyl radical containing 1 to 4 carbon atoms.

4. Process for preparing a product according to one of claims 1, 2 or 3, characterized in that a product of general formula: 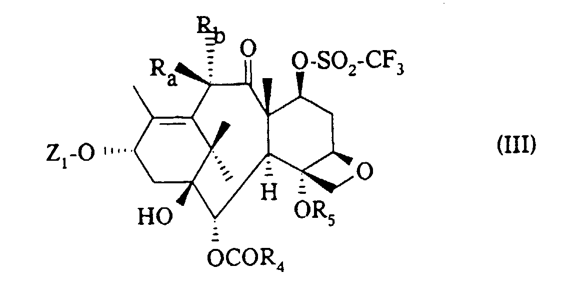 in which Z₁ represents a hydrogen atom or a radical of general formula (II) in which R₁ and R₃ are defined as above or a radical of general formula: 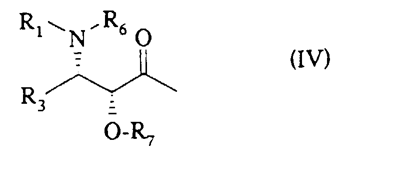 in which R₁ and R₃ are defined as above, and either R₆ represents a hydrogen atom and R₇ represents a group protecting the hydroxyl function, or R₆ and R₇ together form a saturated 5- or 6-membered heterocycle, R₄ and R₅ are defined as above, Rₐ represents a hydrogen atom or an alkoxy, acyloxy or alkoxyacetoxy radical or a protected hydroxyl radical, preferably a 2,2,2-trichloroethoxycarbonyloxy radical, and R_{b} represents a hydrogen atom, or alternatively Rₐ and R_{b}, together with the nitrogen atom to which they are attached, form a keto function, is treated with an alkali metal halide or an alkali metal azide or a quaternary ammonium salt or an alkali metal phosphate to obtain a product of general formula: 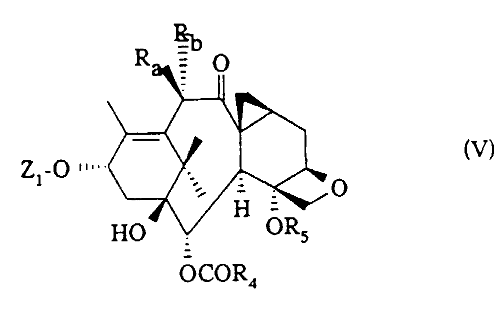 in which Z₁, R₄, R₅, Rₐ and R_{b} are defined as above, followed, if necessary, by replacement of the protective group borne by Rₐ or of the protective groups represented by R₇ and/or by R₆ and R₇ by hydrogen atoms.

5. Process for preparing a product according to one of claims 1 to 3 for which R₄ and R₅ are defined as in one of claims 1 to 3 and Rₐ and R_{b} each represent a hydrogen atom, characterized in that a product according to one of claims 1 to 3 for which Rₐ represents a hydroxyl, acyloxy or alkoxyacetoxy radical is reduced electrolytically.

6. Process for preparing a product according to one of claims 1 to 3 for which R₄ and R₅ are defined as in one of claims 1 to 3 and Rₐ and R_{b}, together with the carbon atom to which they are attached, form a keto function, characterized in that a product according to one of claims 1 to 3, for which Rₐ represents a hydroxyl radical and R_{b} represents a hydrogen atom is oxidized.

7. Pharmaceutical composition, characterized in that it contains at least one product according to one of claims 1 to 3 for which Z represents a radical of general formula (II), in combination with one or more pharmaceutically acceptable products which may be inert or pharmacologically active.
